# NEUE EUROPÄISCHE PATENTSCHRIFT

(11) **EP 2 051 751 B2**
(45) Veröffentlichungstag und Bekanntmachung des Hinweises auf die Entscheidung über den Einspruch: **17.06.2020**
(45) Hinweis auf die Patenterteilung: 21.04.2010
(21) Anmeldenummer: 07785682.1
(22) Anmeldetag: 01.08.2007
(51) Int. Cl.: A61M 1/10

(54) **BLUTPUMPE**
BLOOD PUMP
POMPE À SANG

(30) Priorität: 06.08.2006 DE 102006036948
(43) Veröffentlichungstag der Anmeldung: 29.04.2009
(73) Patentinhaber: Akdis, Mustafa, 35799 Merenberg (DE)
(72) Erfinder: Akdis, Mustafa, 35799 Merenberg (DE)
(74) Vertreter: Patentanwälte Olbricht Buchhold Keulertz
(86) Internationale Anmeldenummer: PCT/DE2007/001374
(87) Internationale Veröffentlichungsnummer: WO 2008/017289

(56) Entgegenhaltungen:
- EP-B1- 1 135 182
- WO-A-98/11650
- WO-A-2004/098677
- WO-A-2004/106746
- WO-A-2005/028000
- WO-A1-2005/028000
- WO-A1-2005/090791
- AU-B2- 760 610
- DE-A1-102004 019 721
- DE-T2- 69 828 926
- US-A- 5 112 200
- US-A- 5 840 070
- US-A- 6 071 093
- US-A- 6 135 710
- US-A1- 2004 091 354
- US-A1- 2005 281 685
- US-B1- 6 227 820
- US-B1- 6 234 772
- US-B1- 6 250 880
- US-B1- 6 293 901
- US-B2- 7 682 301
- Strömungsmaschinen, Grundlagen und Anwendungen, Seite 234, von Herbert Sigloch, 3. Auflage, Hanser Verlag
- Kreiselpumpen, Ein Handbuch für Entwicklung, Anlagenplanung und Betrieb, Seiten 464 und 465, von Johann F. Jülich, Springer Verlag
- Entwicklung und Auslegung von Radial-Axial-Lagerungen für chronisch einsetzbare Rotations-Blutpumpen; Studienarbeit von Thomas Kink; November 1996; Institut für Allgemeine Konstruktionstechnik des Maschinenbaus, Seite 29
- Konzept einer berührungslosen Lagerung für Rotations-Blutpumpen; Dissertation, vorgelegt von Thomas Christoph Kink, Prüfung am 21.02.2005, Seiten VIII, IX, 102, 103, 108, 109, 156, 157
- Visualisierung der Lager-Durchströmung einer Mikro-axial-Blutpumpe; Diplomarbeit von Jan Baumert, Juli 2002, Helmholzinstitut für Biomedizinische Technik

## Beschreibung

Die Erfindung betrifft eine Blutpumpe. Insbesondere betrifft die Erfindung eine Blutpumpe nach dem Oberbegriff des Patentanspruchs 1 zur Förderung von Blut in extrakorporalen Kreisläufen vorzugsweise über eine kurz- bis mittelfristige Zeitdauer (von 6 Stunden bis 6 Monaten) sowie in intrakorporalen Kreisläufen über eine langfristige Zeitdauer (von 6 Monaten bis 10 Jahren).

### Hintergrund der Erfindung:

Während der letzten Jahrzehnte haben sich mechanische Herzunterstützungssysteme immer mehr als therapeutisches Mittel zur Behandlung der chronischen Herzinsuffizienz durchsetzen können. Ihre Hauptaufgabe besteht darin, die Blutzirkulation aufrechtzuerhalten und somit den Sauerstoffbedarf von Organen und Geweben im Falle eines Herzversagens zu gewährleisten. Neuere Entwicklungen im Bereich der mechanischen Kreislaufunterstützungssysteme haben dazu beigetragen, eine Vielfalt von Pumpmechanismen hervorzubringen, die je nach klinischer Indikation von einseitigen Ventrikel-Unterstützungsystemen (engl. VAD = Ventricular Assist Device) bis zum vollständigen Herzersatz bzw. zu Kunstherzsystemen (eng. TAH = Total Arificial Heart) reichen. Es ist in der heutigen Herzchirurgie weitgehend bekannt, dass der klinische Bedarf nach VAD-Systemen wesentlich höher liegt als der Bedarf nach TAH-Systemen, wobei den linksventrikulären Assist-Devices (LVAD) aufgrund der höheren hämodynamischen Belastung der linken Herzhälfte ein besonderer Stellenwert zukommt. Als wesentliches Therapieziel eines Assist-Device ist die "Überbrückung zur Transplantation" (engl. Bridge-to-Transplant) zu nennen, wobei das VAD-System die Pumpleistung des insuffizienten Herzens solange übernimmt bzw. unterstützt, bis ein geeignetes Spenderorgan verfügbar wird und dann eine Herztransplantation durchgeführt werden kann. Einer relevanten Statistik zufolge erreichen ca. 70% der Patienten mit mechanischen Unterstützungssystemen die Herztransplantation, die dann mit einer Sterblichkeit von unter 10 % durchgeführt wird (Hammel et al., Mechanische myokardiale Unterstützungssysteme 1997, Zeitschrift Anaesthesist, Nr. 46, S. 408-418, 1997). Eine weitergehende Behandlung der klinischen und technischen Hintergründe und Besonderheiten der VAD- und TAH Systeme ist in Akdis et al. (Handbuch der Kardiotechnik, 4. Auflage, S. 461-482, Urban & Fischer Verlag, 2002) und Hetzer et al. (Kardiale Assist-Systeme: Gegenwärtiger Stand, Zeitschrift Herz, Nr. 5, 407-417, Urban & Vogel Verlag, 2002) publiziert.

Aus der Druckschrift DE 698 28 926 T2 ist eine Blutpumpe mit einem Laufrad bekannt. Hierbei weist die Blutpumpe einen Einlass und einen Auslass auf. Die radiale Lagerung des Laufrades erfolgt bei dieser Blutpumpe über zwei Magnetlager. Weiterhin wird das Laufrad in radialer Richtung über hydrodynamische Lager in Form von erhöhten Flächen und Kontaktflächen stabilisiert. Diese hydrodynamischen Lager bewirken bei Drehung des Laufrades, dass das Laufrad vom Gehäuse durch einen Flüssigkeitsfilm getrennt wird. Die Funktionalität dieser hydrodynamischen Lager basiert auf verengten Lagerspaltgeometrien, die aus mehreren rechteckig geformten oder keilförmigen Lagerelementen gebildet werden.

Eine ähnliche Blutpumpe ist aus WO 98/11650 bekannt.

Der vorliegenden Erfindung liegt die Aufgabe zugrunde, eine Blutpumpe zu realisieren, die blutschonend betrieben werden kann und einen besonders kompakten Aufbau aufweist. Dies wird in der vorliegenden Erfindung durch eine Vorrichtung zur Förderung von Blut, insbesondere eine Kreiselpumpe erreicht, die in ihrem Aufbau und ihrer Funktionsweise hin sichtlich Pumpendesign, Antriebskonzept, Strömungsführung und insbesondere Rotorlagerung einen blutschonenden Einsatz am Patienten über die jeweilige Einsatzdauer ermöglicht. Erfindungsgemäß wird diese Aufgabe durch eine Blutpumpe nach Anspruch 1 gelöst.

Weitere Vorteile und Besonderheiten der Erfindung ergeben sich aus den Unteransprüchen und der nachfolgenden Darstellung bevorzugter Ausführungsbeispiele anhand der Abbildungen.

Vorteilhafte Weiterbildungen der Erfindung sind Gegenstand der Ansprüche 2 bis 18.

Die Erfindung beinhaltet eine Blutpumpe mit einem Laufrad mit einer Drehachse in einem Pumpengehäuse, wobei die Blutpumpe einen Einlass und einen Auslass aufweist.

Erfindungsgemäß zeichnet sich die Blutpumpe dadurch aus, dass sie mindestens zwei räumlich voneinander getrennte Elemente enthält, wobei wenigstens eines der Elemente so beschaffen ist, dass es ein Lomakin-Lager darstellt und dass mindestens ein weiteres Element ein radiales Magnetlager darstellt.

Es ist vorteilhaft, dass das strömungsmechanisch stabilisierende Element durch das Pumpengehäuse und ein Pumpenlaufrad und/oder einen Bestandteil des Pumpenlaufrades gebildet wird.

Der Begriff strömungsmechanisch stabilisierend beinhaltet insbesondere ein Lager, vorzugsweise ein strömungsmechanisches Radiallager.

Es ist vorteilhaft, dass das radiale Magnetlager ein elektromagnetisches Radiallager ist.

Es ist vorteilhaft, dass das radiale Magnetlager ein permanentmagnetisches Radiallager ist.

Es ist vorteilhaft, dass das radiale Magnetlager mindestens zwei Ringmagnete beinhaltet.

Es ist vorteilhaft, dass wenigstens einer der Ringmagnete in dem Pumpenlaufrad und/oder in einen der Bestandteile des Pumpenlaufrades integriert ist.

Es ist vorteilhaft, dass das radiale Magnetlager durch abstoßende magnetische Kräfte wirkt.

Es ist vorteilhaft, dass das radiale Magnetlager durch anziehende magnetische Kräfte wirkt.

Es ist vorteilhaft, dass das radiale Magnetlager als separates permanentmagnetisches Radiallager ausgebildet ist.

Es ist vorteilhaft, dass das radiale Magnetlager als separates elektromagnetisches Radiallager ausgebildet ist.

Es ist vorteilhaft, dass das radiale Magnetlager einen Rotormagneten im Laufrad und einen Statormagneten im Pumpengehäuse beinhaltet.

Es ist vorteilhaft, dass die magnetische Kupplung eine diagonale Magnetkupplung ist.

Es ist vorteilhaft, dass das radiale Magnetlager geregelte elektromagnetische Kräfte ausübt.

Es ist vorteilhaft, dass das strömungsmechanisch stabilisierende Element zwischen Laufrad und dem Pumpengehäuse wirkt.

Es ist vorteilhaft, dass das strömungsmechanisch stabilisierende Element zwischen Deckscheibe und dem Pumpengehäuse wirkt.

Es ist vorteilhaft, dass das strömungsmechanisch stabilisierende Element zwischen Laufradschaufeln und dem Pumpengehäuse wirkt.

### Beispiele bevorzugter Wirkmechanismen sind:

Radiale strömungsmechanische Stabilisierung, basierend auf hydrostatischen Kräften Entsprechend einem Lomakin-Effekt, zwischen Laufrad und Pumpengehäuse, oder zwischen Deckscheibe und Pumpengehäuse oder zwischen Laufradschaufeln und Pumpengehäuse, und einem Drosselspalt-Effekt, gleichfalls zwischen Laufrad und Pumpengehäuse und/oder zwischen Deckscheibe und Pumpengehäuse und/oder zwischen Laufradschaufeln und Pumpengehäuse.

Weitere Beispiele bevorzugter Wirkmechanismen für radiale strömungsmechanische Stabilisierung basieren auf hydrodynamischen Kräften (Reynolds-Effekt) zwischen Laufrad und Pumpengehäuse und/oder zwischen Deckscheibe und Pumpengehäuse und/oder zwischen Laufradschaufeln und Pumpengehäuse oder basieren auf hydraulischen Kräften zwischen Laufrad und Pumpengehäuse und/oder zwischen Deckscheibe und Pumpengehäuse und/oder zwischen Laufradschaufeln und Pumpengehäuse.

Es ist vorteilhaft, dass die Blutpumpe eine Axiallagerung des Laufrades aufweist.

Es ist vorteilhaft, dass die Axiallagerung des Laufrades auf permanentmagnetischen Kräften basiert.

Es ist vorteilhaft, dass die Axiallagerung des Laufrades auf elektromagnetischen Kräften basiert.

Es ist vorteilhaft, dass die Axiallagerung des Laufrades auf strömungsmechanischen Kräften basiert.

Beispiele für die Axiallagerung des Laufrades sind:
**a. Magnetisches Axiallager**
   **i. basierend auf permanentmagnetischen Kräften**
      1. separates permanentmagnetisches Axiallager (Rotormagnet im Laufrad, Statormagnet im Pumpengehäuse)
         a. abstoßende magnetische Kräfte
            i. ringförmige Äusführung von Rotor- und Statormagnet
            ii. axial versetzte Anordnung von Rotor- und Statormagnet
         b. anziehende magnetische Kräfte
            i. ringförmige Ausführung von Rotor- und Statormagnet
            ii. axial versetzte Anordnung von Rotor- und Statormagnet
      2. in der permanentmagnetischen Kupplung integriertes Axiallager
         a. basierend auf einer radialen Magnetkupplung
         b. basierend auf einer diagonalen Magnetkupplung
   **ii. basierend auf elektromagnetischen Kräften**
      1. geregelte magnetische Kräfte
         a. Aktiv Magnetlager (Statormagnet im Gehäuse, Rotormagnet im Laufrad)
            i. basierend auf einem radialen Aktiv-Magnetlager
            ii. basierend auf einem diagonalen Aktiv-Magnetlager
      2. ungeregelte elektromagnetische Kräfte
         a. elektromagnetische Kupplung
            i. basierend auf einer axialen Magnetkupplung
            ii. basierend auf einer diagonalen Magnetkupplung
**b. Strömungsmechanisches Axiallager (zwischen Laufrad und Pumpengehäuse)**
   1. hydrodynamisches Axiallager
**c. Mechanisches Axiallager (zwischen Laufrad und Pumpengehäuse)**
   1. Pivot-Lager (Spitzenlager oder Kugelspurlagerung)

Zweckmäßigerweise zeichnet sich das Pumpendesign durch eine harmonische Flachbauweise, vorzugsweise mit elliptischem Querschnitt des Pumpengehäuses und/oder des Elektromotors aus.

Beispiele für bevorzugte Antriebskonzepte sind:
- A) Blutpumpe nach einem der vorangehenden Punkte, wobei die Blutpumpe als trennbarer Pumpenkopf ausgeführt ist und über eine externe (wieder verwendbare) Antriebseinheit angetrieben wird
- B) Blutpumpe nach A), wobei die Antriebseinheit im Wesentlichen einen Elektromotor und/oder eine magnetische Kupplungsvorrichtung beinhaltet
- C) Blutpumpe und oder Verfahren nach A), wobei die Antriebseinheit eine über Hochdruckgas angetriebene Turbine darstellt
- D) Blutpumpe und/oder Verfahren nach C), wobei Hochdrucksauerstoff als Hochdruckgas verwendet wird
- E) Blutpumpe und/oder Verfahren nach D), wobei Sauerstoff gleichzeitig für eine räumlich getrennte Vorrichtung zur Blutoxygenierung verwendet wird.

### Hydrostatische Radiallagerung und Hydrodynamische Axiallagerung:

Es ist zweckmäßig, dass mindestens eins der elektromagnetischen Elemente ein hydrostatisches Radiallager darstellt.

### Mechanisch-Magentische Rotorlagerung:

Es ist zweckmäßig, dass die Blutpumpe mit einem Schaufelrad mit einer Drehachse in einem Pumpengehäuse mit einem Zuströmkanal und mit mindestens einer mechanisch berührenden Lagervorrichtung und mindestens einer permanentmagnetischen Vorrichtung so ausgestaltet ist, dass mindestens eine mechanisch berührende Vorrichtung eine Kugelspurlagerung und mindestens eine permanentmagnetische Vorrichtung ein in radialer Richtung instabiles und in axialer Richtung stabiles permanentmagnetisches Lager darstellt.

Es ist zweckmäßig, dass das permanentmagnetische Lager im Wesentlichen auf zwei Ringmagneten beruht, die zueinander gegensinnig polarisiert sind.

### Harmonische Flachbauweise:

Es ist zweckmäßig, dass das Höhen-Breitenverhältnis des Pumpengehäuses kleiner als 1 ist.

### Trennbarer Pumpenkopf mit elektromagnetischem Antrieb:

Es ist zweckmäßig, dass die Blutpumpe von der Antriebseinheit getrennt werden kann und die Antriebseinheit wieder verwendbar ist.

### Trennbarer Pumpenkopf mit pneumatischem (O₂-) Turbinen-antrieb:

Es ist zweckmäßig, dass zur extrakorporalen Förderung von Blut und Sauerstoff, bestehend aus mindestens einer Blutpumpe, mindestens eine Hochdrucksauerstoff- Turbine eingesetzt wird, die räumlich von der Blutpumpe trennbar ausgeführt ist. Vorteilhafterweise wird hierbei die Blutpumpe über eine Turbine angetrieben.

Es ist zweckmäßig, dass die Turbine über das Hochdruck-Sauerstoffreservoir angetrieben, die Blutpumpe über die Turbine angetrieben wird, das Hochdruck-Sauerstoffreservoir den Oxygenator mit Sauerstoff versorgt und die Blutpumpe Blut durch den Oxygenator befördert

Ingenieurwissenschaftlich gesehen, lassen sich die VAD-Systeme im Hinblick auf den wirksamen Pumpmechanismus in zwei Hauptkategorien untergliedern:
1) Verdrängerblutpumpen
2) Kreiselblutpumpen.

Verdrängerblutpumpen (engl. displacement blood pump) bilden die erste Generation von Blutpumpen zur Herzunterstützung und sind im Wesentlichen einer Membranpumpe vergleichbar. Durch die pulsatile Volumenänderung einer blutgefüllten Arbeitskammer mit Hilfe pneumatischer, hydraulischer und/oder elektromechanischer Zusatzenergie wird ähnlich zu einer Kolbenpumpe ein physiologischer Druck und Flow aufgebaut, welcher Blut aus dem linken Ventrikel ansaugt und in die Aorta fördert. Der wesentliche Vorteil dieser Systeme liegt in ihrer pulsatilen und somit dem natürlichen Herzen nachgeahmten Arbeitsweise, wohingegen ihr großer Bauraum problematisch für eine Implantation der Pumpe sowie aufgrund ihrer komplexen Funktionsweise fertigungsaufwendig ist.

Kreiselblutpumpen (engl. rotary blood pump) gewinnen daher aufgrund ihres einfachen Aufbaus und ihrer minimalen Baugröße zunehmend an klinischer Bedeutung. Im Gegensatz zu Verdrängerblutpumpen liefern die Kreiselblutpumpen keinen pulsatilen, sondern kontinuierlichen Blutfluss durch die Pumpe, was jedoch im Hinblick auf die physiologische Akzeptanz in Fachkreisen als vertretbar auch für den Langzeiteinsatz über mehrere Jahre beurteilt wurde (Schmid et al., Chirurgische Therapieoptionen bei schwerer Herzinsuffizienz, Deutsches Ärzteblatt, Jg. 101, Heft 7, S. 429-435).

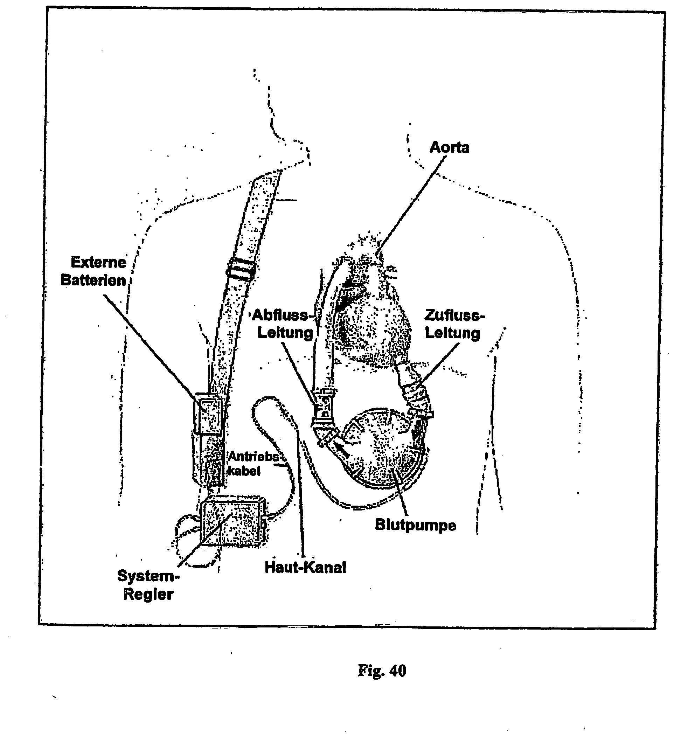

Der wesentliche Aufbau eines Kreiselblutpumpensystems umfasst ein drehbar gelagertes Laufrad (Rotor), welches in der Regel über einen elektrischen Motor mit entsprechender Kupplungsvorrichtung angetrieben wird und die ihm zugeführte Rotationsenergie in hydraulische Energie zum Druck- und Flussaufbau verwandelt, sowie ein die Pumpenkomponenten gegen die Umgebung abgrenzendes Pumpengehäuse. Zum Weiterleiten des Förderfluides (Blut) zwischen der Blutpumpe und den jeweiligen Blutgefäßen werden Kanülen und Grafts verwendet, die am Ein- und Auslass der Pumpe befestigt werden müssen. Weiterhin können in einer separaten Controler-Unit die elektronischen Steuer- und Reglerelemente zur Ansteuerung der Pumpe sowie Sensoren zur Überwachung der Pumpparameter untergebracht werden. Für mobile Einsätze der Pumpe bietet sich zudem die Möglichkeit, die Energieversorgung über tragbare Batterien zu gewährleisten. In der Abbildung Fig. 40 ist schematisch der VAD-Einsatz einer Blutpumpe und ihrer Zubehörkomponenten wiedergegeben.

Im Hinblick auf eine einfache Handhabung und einen patientenschonenden Betrieb beim klinischen Einsatz kommt weiterhin der Platzierung der Blutpumpe relativ zum Körper des Patienten ein hoher Stellenwert zu. Prinzipiell lässt sich festhalten, dass eine Blutpumpe je nach klinischer Anwendung entweder außerhalb des Körpers (extrakorporal) oder aber auch innerhalb des Körpers (intrakorporal) platziert wird. Beim ersten Ansatz unterscheidet man weiterhin nach jenen Blutpumpensystemen, die aufgrund der Kompaktheit der Blutpumpe in der Nähe des Patienten (parakorporal) platziert werden können. Bei der intrakorporalen Platzierung der Blutpumpe unterscheidet man hingegen zwischen jenen Systemen, die unter der Haut (subkutan), im Brustkorb (intrathorakal), unterhalb des Zwerchfells (subdominal), innerhalb eines Blutgefäßes (intravasal) oder aber innerhalb der Herzkammern selber (intrakardial, intraventrikulär) platziert sein können.

Es ist anzumerken, dass insbesondere für kurzfristige Applikationszeiten, z.B. in einer Herz-Lungen-Maschine (HLM) oder bei der extrakorporalen Membranoxygenation (ECMO) sowie auch für kurz- bis mittelfristige Einsätze als VAD-System über eine Zeitdauer von bis zu 6 Monaten die Kreiselblutpumpe in der Regel extrakorporal platziert wird, während für längerfristige Applikationszeiten die Blutpumpe aufgrund des Infektionsrisikos offener Wunden vorrangig innerhalb des Körpers untergebracht ist. Die genauere Platzierung der Pumpe im Körper hängt dann im Wesentlichen von der klinischen Indikation sowie insbesondere der Größe und den weiteren technischen Eigenschaften der Blutpumpe ab. Allgemeines Ziel bei der Entwicklung einer implantierbaren Kreiselblutpumpe ist es dennoch, die Baugröße (vorgegeben durch die Hauptabmessungen des Pumpengehäuses) weitgehend zu minimieren, um somit den Kontakt von Blut und Geweben mit körperfremden Oberflächen auf das Minimale zu reduzieren.

Aufgrund der Drehbewegung des Laufrades werden Kreiselblutpumpen und insbesondere deren Pumpengehäuse vorrangig in kreiszylindrischer Form gebaut, wobei der Durchmesser des Laufrades im Wesentlichen die Hauptabmessung der Kreiselblutpumpe bestimmt. Ein anatomiekonformes Designs für verschiedene klinische Indikationen und Implantationspositionen der Pumpe kann dennoch nicht ausreichend mit einer kreiszylindrischen Außenform der Pumpe abgedeckt werden, und es besteht zunehmend der klinische Bedarf nach einem der lokalen Anatomie des Menschen angepassten Pumpendesign.

Der vorliegenden Erfindung liegt ferner die Aufgabe zugrunde, eine Blutpumpe zu realisieren, welche in ihrem Design die Implantation in verschiedenen Bereichen des menschlichen Körpers ermöglicht.

Des Weiteren stehen den Vorteilen beim Einsatz einer Kreiselblutpumpe gewisse Gefahren und Risiken gegenüber, die in den Besonderheiten des physiologischen Mediums Blut als Fördermittel begründet sind. Vorrangig ist an dieser Stelle die Schädigung von korpuskularen Blutbestandteilen wie Erythrozyten und Thrombozyten zu nennen, welche auf die in der Kreiselpumpe induzierten Strömungs- und Temperaturfelder sowie der Interaktion des Blutes mit technischen Oberflächen zurückzuführen ist. Zu hohe Scherspannungen auf Erythrozyten können beispielsweise zur Folge haben, dass deren Zellwand beschädigt wird und somit die Hämolyserate übermäßig erhöht wird. Andererseits gilt es auch, Strömungsarmut und Strömungsrezirkulationen zu vermeiden sowie die Temperatur der blutkontaktierenden Pumpenkomponenten innerhalb der Grenzen physiologischer Vertretbarkeit (Bluttemperatur ca. 37° C) zu halten, da bei stagnierender Strömung oder zu großer Erwärmung des Blutes die Gefahr der Thrombozytenaktivierung und der daraus resultierenden Gerinnselbildung sowohl eine Gefahr für den Patienten (Infarkte und Embolien aufgrund von Verstopfung peripherer Blutgefäße) als auch das Risiko eines Pumpenausfalls einhergeht. Allgemeine Richtlinie bei der Entwicklung einer Kreiselblutpumpe ist es daher, die Durchströmung der Pumpe effektiv durch Vermeidung von stagnierenden oder rezirkulierenden Strömungsgebieten (Totwassergebieten) zu gestalten sowie die Scherspannungen und Temperaturerhöhungen im Blut weitgehend zu minimieren. Lokale Gefahrenzonen im Sinne von Hämolyse und Thrombogenität sind daher die engen Seitenräume zwischen Laufrad und dem Gehäuse sowie insbesondere die Rotorlagerung des Laufrades. Die Besonderheiten der Rotorlagerung einer Kreiselblutpumpe im Gegensatz zu konventionellen Kreiselpumpen liegen wiederum in der Besonderheit des Mediums Blut, sodass vielfach die bewährten Rotorlagervarianten des klassischen Maschinenbaus nicht ohne weiteres für den Einsatz in einer Blutpumpe geeignet sind. Darüber hinaus muss bei Realisierung der Rotorlagerung einer Blutpumpe auch der damit verbundene Aufwand berücksichtigt werden.

In Kreiselblutpumpen liegen eine Reihe von Rotorlagerungskonzepten vor, die folgendermaßen untergliedert werden können:
1) Kugellager mit Wellendichtungen zur Abdichtung der Lager gegen das Blut
2) Gleitlager in Blut
3) Berührungslose Magnetlager

Der Vorteil der ersten Variante liegt in ihrem einfachen Aufbau, wohingegen ihre kurze Lebensdauer aufgrund Leckage an der Dichtung den Langzeiteinsatz in einer Blutpumpe nicht ermöglicht. Die Gleitlager hingegen zeigen zumindest eine für mittelfristige Applikationen ausreichende Lebensdauer, müssen jedoch in ihrem Design dahingehend optimiert werden, dass die tribologischen Verhältnisse (Lagerreibung und Erwärmung) nicht zu einer Schädigung des Blutes führen. Die Magnetlager schließlich bieten den wesentlichen Vorteil, berührungslos und damit reibungsfrei zu arbeiten, sind jedoch aufgrund des hohen Herstellungsaufwandes für die elektromagnetischen Komponenten des Magnetlagers nicht für kurzfristige Anwendungen geeignet.

Aufbau und Funktionsweise der Blutpumpe :

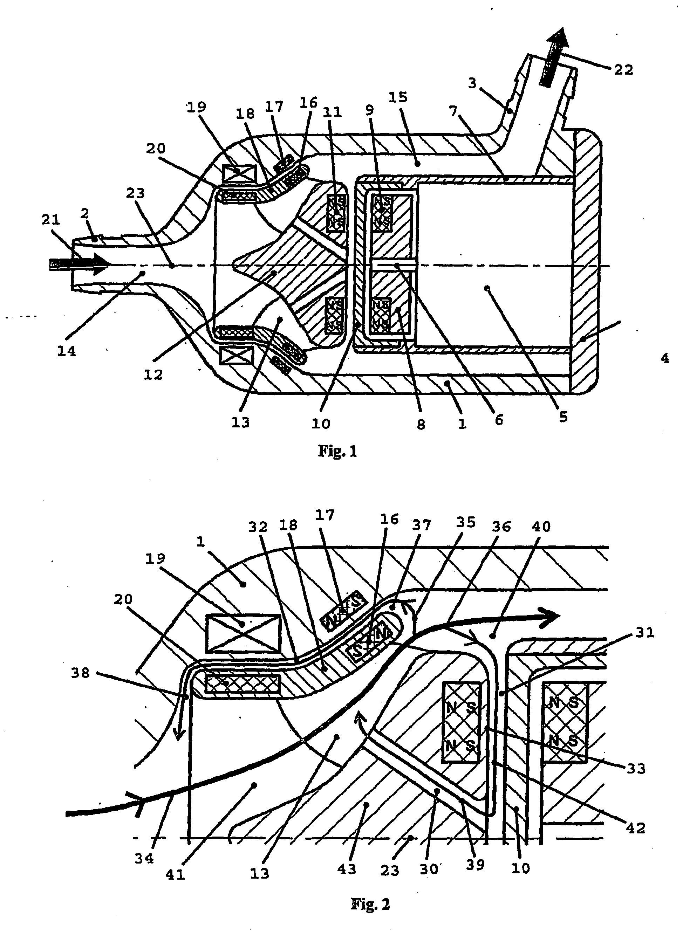

Der Aufbau und die Funktionsweise der Blutpumpe gemäß der Erfindung sei beispielhaft anhand der Abbildung Fig. 1 und Fig. 2 näher erläutert.

In ihrer Gesamtheit besteht die Blutpumpe aus den folgenden 5 Hauptkomponenten:
1) Pumpengehäuse
2) Antriebseinheit
3) Pumpenlaufrad
4) Rotorlagerung
5) Strömungsführung

Im Rahmen der Beschreibung kommen insbesondere der Rotorlagerung des Pumpenlaufrades im Pumpengehäuse eine zentrale Rolle zu, sodass die Rotorlagerung im abschließenden Teil bei der Beschreibung näher erläutert wird.

### Pumpengehäuse :

Das Pumpengehäuse (1) besitzt im Wesentlichen eine hohlzylindrische Form und umschließt als übergeordnete Einheit in ihrem Innenraum die weiteren Komponenten der Blutpumpe. Bei Blutpumpen mit externem Antrieb (d.h. Antrieb ist außerhalb des Innenraumes des Pumpengehäuses positioniert) umschließt die Blutpumpe lediglich das Pumpenlaufrad, ihre Rotorlagerung sowie die Strömungsführung in der Pumpe. Im Rahmen der vorliegenden Erfindung werden sowohl Blutpumpen mit außerhalb liegendem Antrieb als auch jene Pumpen mit integriertem Antrieb in Betracht gezogen. Die Abbildungen zu der Erfindung sowie deren Beschreibungen geben weitergehende Auskünfte zu dieser Thematik.

Das Pumpengehäuse (1) ist an einem Ende mit dem Pumpeneinlass (2) sowie an einem anderen Ende mit dem Pumpenauslass (3) fest verbunden. Ein Pumpendeckel (4) verschließt im Falle eines integrierten Antriebs den Innenraum des Pumpengehäuses (1) gegen die Umgebung.

### Antriebseinheit :

Die Antriebseinheit dient im Wesentlichen dazu, die Drehbewegung des Laufrades aufrechtzuerhalten. Im gegenwärtigen Stand der Technik werden für Blutpumpen ausschließlich Antriebe verwendet, die eine elektrische Energiequelle haben und somit als Elektromotoren ausgeführt sind. Im Rahmen der vorliegenden Erfindung liegt eine Besonderheit darin, dass ein verfahren und eine Vorrichtung zur Förderung von Blut vorgestellt wird, welche als Antrieb nicht über elektrische Energie, sondern pneumatische Energie im Zusammenwirken mit einer Turbine versorgt wird. Dieses Konzept macht insbesondere bei all jenen Anwendungen von Blutpumpen einen effektiven Sinn, wo die Turbine über eine Hochdruck-Sauerstoffversorgung angetrieben wird, und Sauerstoff in jedem OP-Raum und in jeder Klinik-Station verfügbar ist. Des Weiteren lässt sich durch Kombinieren einer Blutpumpe mit einem Blutoxygenationssystem (Oxygenator) ein geeigneter Synergie-Effekt nutzen, indem Sauerstoff sowohl für die Oxygenation als auch für den Pumpenantrieb verwendet werden kann. Insbesondere für kurz- bis mittelfristige Anwendungen (Herz-Lungen-Maschine, ECMO, etc.) bei extrakorporaler Platzierung der Blutpumpe bringt ein derartiges Antriebskonzept im Vergleich zu Elektromotoren wesentliche Vorteile hinsichtlich der Aufwandsreduzierung.

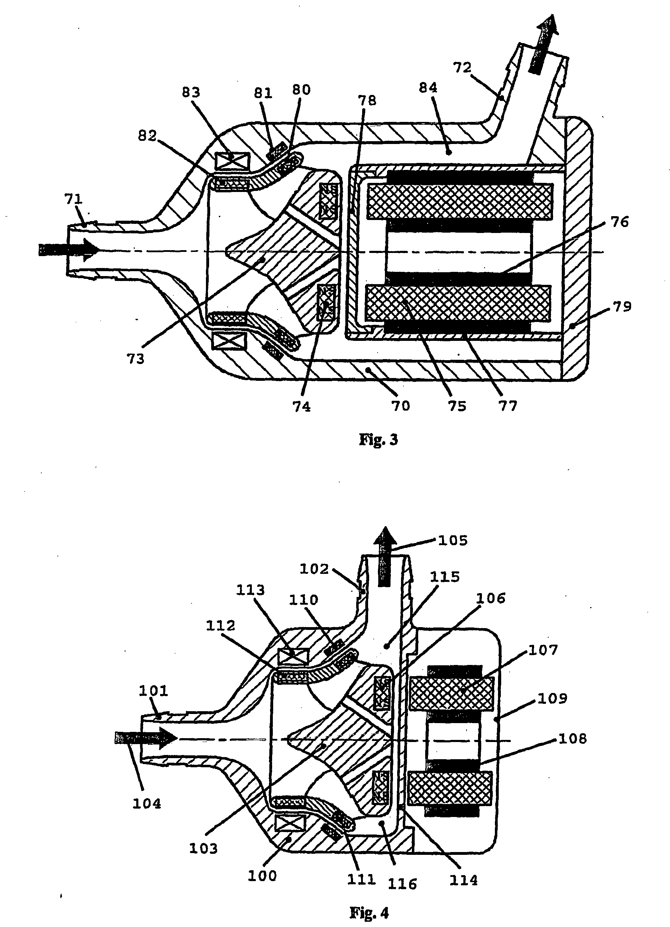

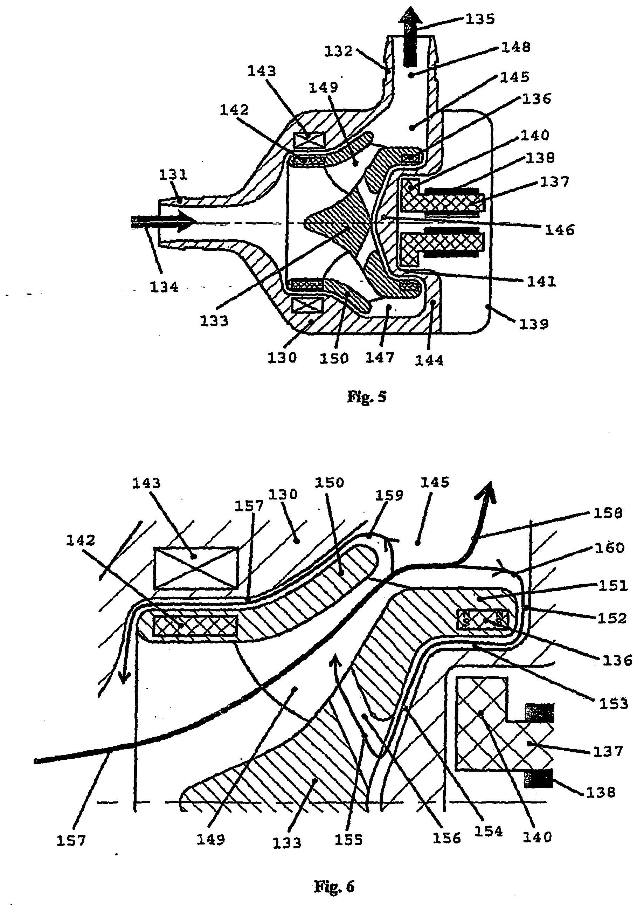

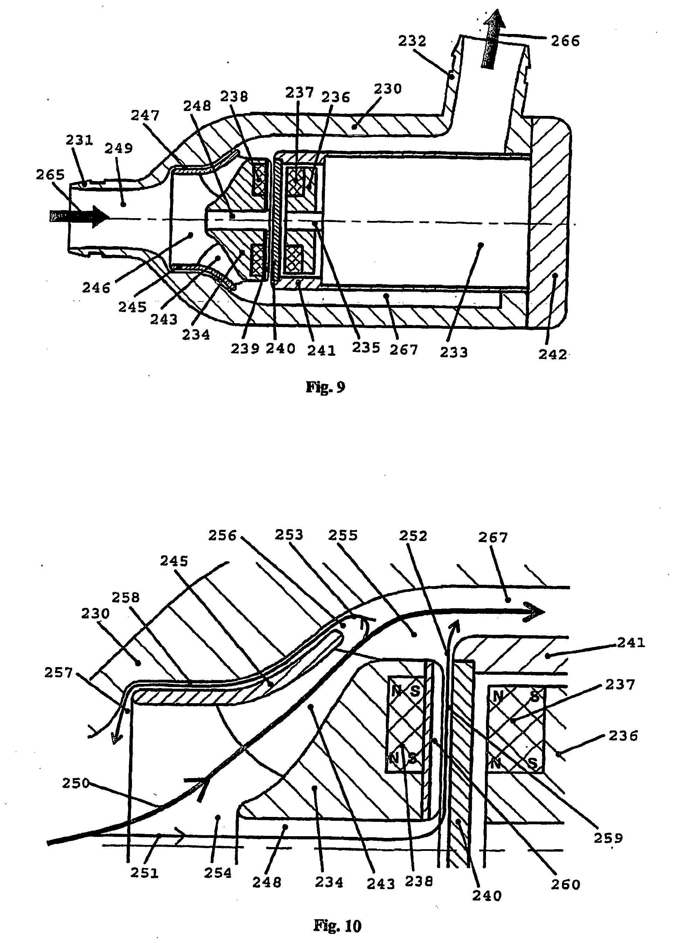

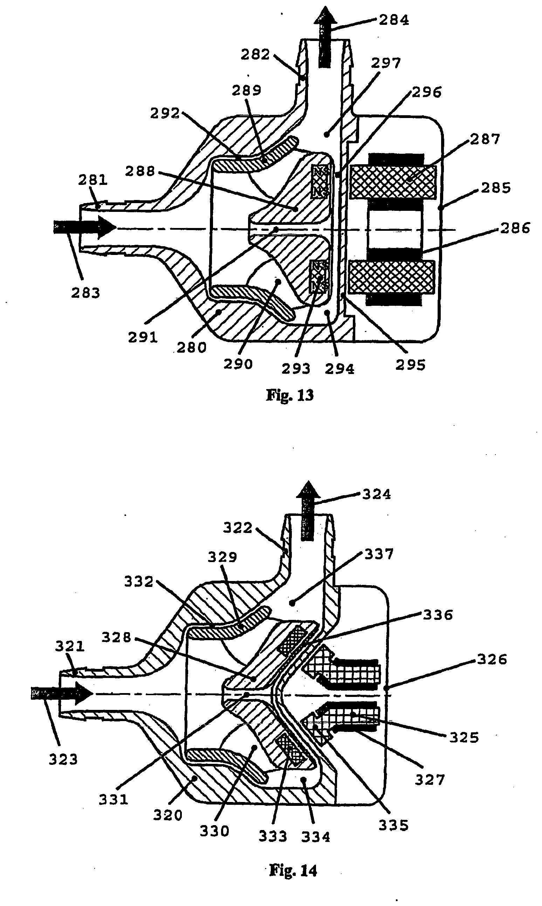

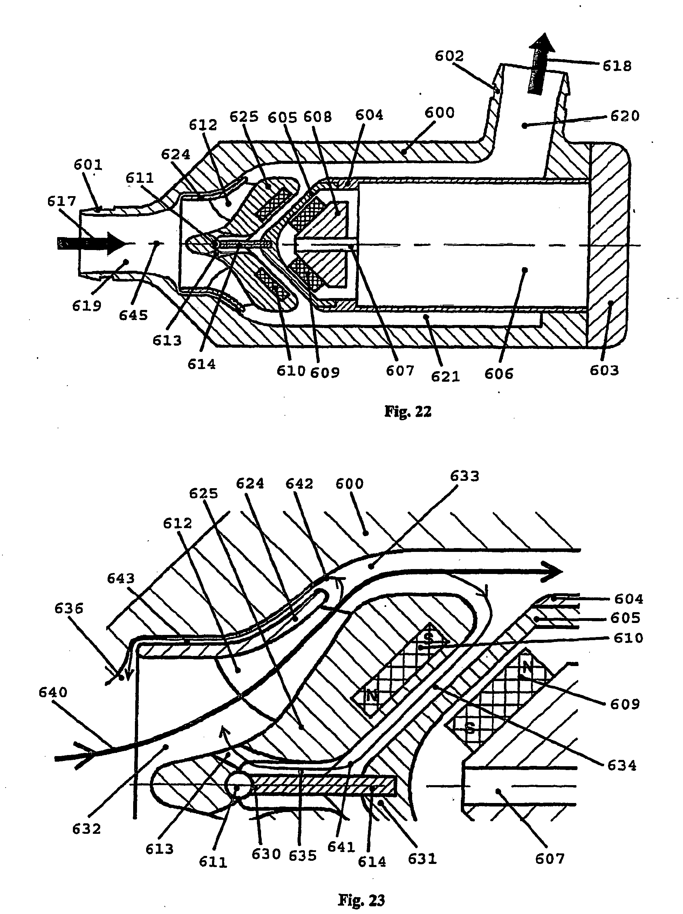

Für implantierbare Anwendungen sind dennoch Blutpumpen mit elektromagnetischem Antrieb die geeignetere Antriebsvariante und sollen im Folgenden anhand von Fig. 1 und Fig. 2 näher erläutert werden. Die Antriebseinheit (5, 6, 7, 8, 9) besteht zum Wesentlichen aus einem Elektromotor (5) sowie einer für die Übertragung der Drehbewegungen vom Motor (5) auf das Pumpenlaufrad (12) erforderlichen Kupplungsvorrichtung (8, 9). Es werden sowohl elektromagnetische Antriebe (elektromagnetische Kupplung) als auch Elektromotoren mit permanentmagnetischer Kupplung in Erwägung gezogen. Eine Blutpumpe mit permanentmagnetischer Kupplung ist beispielsweise in Fig. 1 und Fig. 9 wiedergegeben. Eine Blutpumpe mit elektromagnetischer Kupplung ist beispielsweise in Fig. 3 und Fig. 13 wieder zu finden. Es werden die in der Kupplungsvorrichtung wirksamen Kräfte und Momente je nach Wirkrichtung der Kupplung (axial, radial, diagonal) zur Stabilisierung des Laufrades im Pumpengehäuse und damit zur Rotorlagerung genutzt. Eine axial wirkende Magnetkupplung ist beispielsweise in Fig. 1 zu ersehen. Bei einer Axialkupplung wirken die magnetischen Anziehungskräfte zwischen den Antriebsmagneten (9) und Abtriebsmagneten (11) in axialer Richtung. Eine radial wirkende Magnetkupplung ist beispielsweise in Fig. 5 dargestellt. Die magnetischen Anziehungskräfte in der Kupplungsvorrichtung (140, 136) wirken hierbei in radialer Richtung. Bei einer diagonal wirkenden Magnetkupplung, wie dies beispielsweise in Fig. 22 verdeutlicht ist, wirken die magnetischen Anziehungskräfte zwischen den Antriebsmagneten (609) und den Abtriebsmagneten (610) in einer diagonalen Richtung (Mischform aus axial und radial).

Die vorliegende Erfindung beinhaltet weiterhin eine für subkutane Implantationen geeignete Flachbauweise der Blutpumpe, bei dem das Pumpengehäuse derart gestaltet ist, dass der vorhandene Bauraum effektiv durch die Antriebseinheit ausgenutzt wird. Im Rahmen der vorliegenden Erfindung werden darüber hinaus auch Blutpumpen vorgestellt, die als Antriebssystem eine sauerstoffbetriebene Turbine verwenden und somit insbesondere für kurz- bis mittelfristige Einsatzzeiten ein einfach herstellbares Blutpumpensystem hervorbringen.

### Pumpenlaufrad :

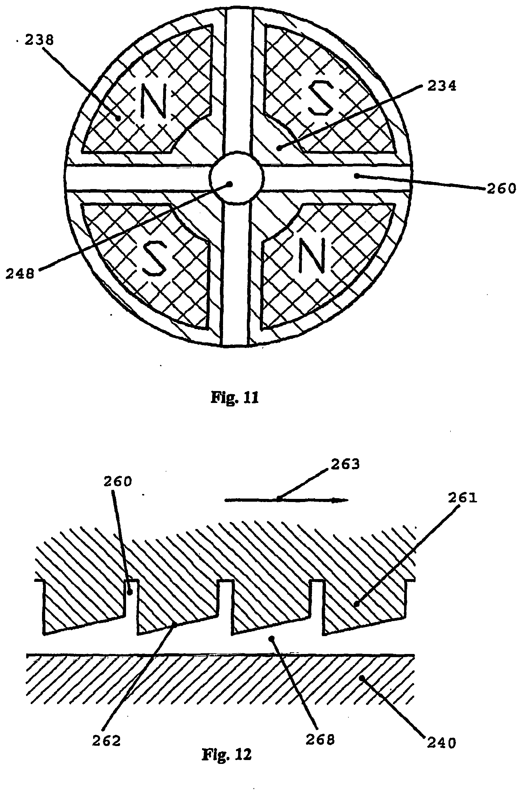

Das Pumpenlaufrad dient im Wesentlichen dazu die ihm über die Kupplung zugeführte Rotationsenergie in hydraulische Energie zum Druck- und Flussaufbau umzuwandeln, welches gleichermaßen der Hauptfunktion der Blutpumpe entspricht. Als Laufräder können in Blutpumpen sowohl Schaufelräder als auch viskos fördernde Rotationskörper zum Einsatz kommen. Da die vorliegende Erfindung wiegend auf beschaufelten Laufrädern beruht, soll im Folgenden unter Laufrad ein Schaufelrad verstanden werden. Je nach Strömungsrichtung innerhalb der Laufradschaufeln unterscheidet man zwischen axialen, radialen und diagonalen Laufrädern. In der vorliegenden Erfindung werden vorwiegend radiale und diagonale Laufräder betrachtet. Der Aufbau und die Funktionsweise des Laufrades sei beispielhaft anhand von Fig. 1 und Fig. 2 näher erläutert. Das Pumpenlaufrad (12) stellt einen mit Schaufeln (13) versehenen Rotationskörper dar, welches Drehbewegungen um ihre Drehachse (23) ausführen kann. Die Übertragung der Drehmomente vom Antrieb auf das Laufrad (12) erfolgt bei der Blutpumpe elektromagnetisch mit Hilfe des Elektromotors (5) und der auf der Motorwelle (6) befestigten Magnetkupplung (8, 9). Hierzu sind antriebsseitig auf dem Polschuh (8) eine gerade Anzahl von wechselweise polarisierten Magnetsegmenten (9) befertigt, die den im Laufrad (12) befindlichen abtriebsseitigen Magnetsegmenten (11) in magnetisch anziehender Ausrichtung gegenüberstehen (siehe Fig. 11). Infolge der Anziehungskräfte zwischen den Abtriebsmagneten (11) und den Antriebsmagneten (9) werden die Drehbewegungen des Motors (5) auf das Laufrad (12) übertragen. Die Rotation des Laufrades (12) im Pumpengehäuse (1) ist gleichermaßen Ursache für den Fluss- und Druckaufbau in der Blutpumpe.

Je nach klinischer Anwendung und Baugröße der Pumpe liegt die Drehzahl des Laufrades (12) zwischen 2000 und 20.000 min-1. Hierbei fördert die Blutpumpe je nach physiologischen Bedürfnissen einen Volumenstrom zwischen 0,5 bis 10 L/min und liefert dabei einen physiologischen Druckaufbau zwischen 0 bis 800 mmHg. Für eine vollständige Entlastung des Herzens eines herzinsuffizienten Patienten ist ein Blutfluss von ca. 5 L/min bei einem physiologischen Druckaufbau von 100 mmHg erforderlich. Der Fluss- und Druckaufbau in der Blutpumpe basiert auf den zentrifugalen Effekten des rotierenden Laufrades (12) im Pumpengehäuse (1). Neben dem Hauptförderstrom (34) werden hierbei eine Reihe von Sekundärströmungen hervorgerufen, denen im Sinne einer blutschonenden Förderung eine große Bedeutung zukommt und die im Folgenden näher erläutert werden.

### Strömungsführung :

Unter Strömungsführung ist hier in erster Linie die Blutströmung durch die Pumpe zu verstehen.

Bei Blutpumpen gelten allgemein die Anforderungen an eine hämatologisch günstige Strömungsführung innerhalb der Pumpe, gekennzeichnet durch:
- die Vermeidung von hohen Scherspannungen in der Strömung (→ Hämolysegefahr)
- die Vermeidung von zu hohen Fluid-Temperaturen (< 42 °C) und zu geringen Strömungsgeschwindigkeiten (→ Gerinnungsgefahr bzw. Thrombengefahr)
- die Vermeidung von Strömungsablösung und Strömungsrezirkulationen (→ Thromben- und Hämolysegefahr)
- die Minimierung des Blutkontaktes mit Fremdoberflächen (materialbedingte Thrombozytenaktivierung bzw. Thrombengefahr)

Anhand von Fig.1 und Fig. 2 sei die Strömungsführung innerhalb der Blutpumpe im Folgenden näher erläutert. Innerhalb der Blutpumpe lässt sich die Strömungsführung prinzipiell untergliedern in Hauptströmung und Sekundärströmungen. Die Hauptströmung (34) ist jene Strömung, die über den Pumpeneinlass (2) in die Pumpe einströmt, über einen den Pumpeneinlass (2) und das Laufrad (12) verbindenden Zuströmkanal (14) zum Laufrad (12) weitergeleitet wird, von dort aus durch die Rotation des beschaufelten Laufrades (12) mit Drall beaufschlagt und befördert wird und schließlich über einen dem Laufradbereich angegliederten Strömungskanal (15) die Pumpe wieder über den Pumpenauslass (3) verlässt. Der Pumpeneinlass (2) ist hierbei mit den Ansauggefäßen (linker Ventrikel bzw. linker Vorhof) des Patienten verbunden, wohingegen der über den Pumpenauslass (3) austretende Blutfluss (22) über entsprechende Verbindungsleitungen (Kanülen, Grafts) an die Aorta (Hauptschlagader) des Patienten befördert wird.

Als Sekundärströmungen sind all jene Strömungsformen in der Blutpumpe zu verstehen, die von der Hauptströmung (34) abzweigen und von dort aus weitere Zwischenräume der Pumpe durchströmen. Insbesondere die Druckverteilung am Laufrad (12), gekennzeichnet durch den höheren Druck am Laufradaustritt (40) gegenüber dem Druck am Laufradeintritt (41), hat zur Folge, dass eine Reihe von sekundären Strömungen (35, 39, 42) hervorgerufen wird, der im Rahmen der vorliegenden Erfindung eine zentrale Rolle zukommt.

Der radiale Spalt (32) zwischen Laufrad (5, 18) und Pumpengehäuse (1) beispielsweise wird aufgrund des höheren Druckes am Laufaustritt (40) von der Einmündung des Spaltraumes (37) zum gegenüberliegenden Niederdruckende (38) des Spaltraumes (32) in vorwiegend axialer Richtung retrograd zur Richtung des Hauptstroms (34) durchströmt und bringt somit eine Leckageströmung (35) hervor. Die Trennung der Leckageströmung (35) von der Hauptströmung (34) erfolgt hierbei über eine der äußeren Umrandung der Schaufelkontur angepasste Deckscheibe (18), die mit den Laufradschaufeln fest verbunden ist. Auf die Besonderheiten der Leckageströmung (35) und ihre strömungsmechanischen Wechselwirkungen mit der Deckscheibe (18) wird im Zusammenhang der Funktionsweise der Rotorlagerung näher eingegangen.

Der axiale Spalt (31) zwischen der Rückseite des Laufrades (5) und dem gegenüberliegenden stationären Pumpenelement (10) bringt einen weiteren Spaltraum (31) hervor, den es im Sinne einer blutschonenden Förderung gilt, frei von Strömungstagnationen zu gestalten.

Eine Möglichkeit zur Auswaschung des axialen Spaltraums (31) ist in Fig. 1 und Fig. 2 dargestellt. Eine bestimmte Anzahl (bevorzugt 2 oder 4) von Spülkanälen (30) ist derart in den Laufradkörper (43) eingearbeitet, dass infolge der Druckverteilung am Laufrad eine Spülströmung (39, 42) vom Hochdruckgebiet am Laufradaustritt (40) zum Niederdruckgebiet auf der Laufradvorderseite (41) induziert wird, welche den axialen Spaltraum (31) auf der Laufradrückseite (33) radial nach innen durchströmt und von dort über die Spülkanäle (30) wieder zurück in die Hauptströmung (34) geleitet wird. Über die Verwendung dieser Strömung insbesondere wird im Zusammenhang der Funktionsweise der Rotorlagerung näher eingegangen.

Eine weitere Variante zur Auswaschung der kritischen Laufradrückseite besteht in der Ausführung der Pumpe gemäß Fig. 9 und Fig. 10, wobei mit Hilfe eines in die Laufradnabe (234) eingearbeiteten zentralen Spülkanals (248) sowie einer auf der Laufradrückseite vorgesehenen sekundären Beschaufelung (260) eine weitere Zentrifugalströmung (252) derart induziert wird, dass über den Spülkanal (248) ein Teil der Hauptströmung (250) als Spülströmung (251, 252) abgezweigt wird und infolge der in den Sekundärbeschauflungen (260) wirksamen Zentrifugalkräften im Seitenraum (259) radial nach außen befördert wird, bis schließlich im Hochdruckgebiet am Laufradaustritt (255) die Spülströmung (252) wieder in die Hauptströmung (250) mündet. Auf die Besonderheiten dieser Spülströmung (251, 252) sowie deren Bedeutung für die erfinderischen Neuheiten im Hinblick auf die Lagerung des Laufrades (234) wird im Zusammenhang der Rotorlagerung näher eingegangen.

### Beschreibung der Erfindung:

Der vorliegenden Erfindung liegt die Aufgabe zugrunde, eine Blutpumpe zu realisieren, die für die Förderung von Blut in extrakorporalen und intrakorporalen Kreisläufen über kurzbis mittelfristige Einsatzdauern (wenigen Stunden bis mehreren Monaten) sowie über langfristige Einsatzdauern (mehreren Monate bis mehreren Jahre) geeignet ist. Dies wird in der vorliegenden Erfindung durch eine Kreiselblutpumpe mit langzeitstabiler und hämokompatibler Rotorlagerung erreicht.

Für den langfristigen Einsatz, bei dem eine Implantation der Blutpumpe im Patienten erforderlich ist, werden im Rahmen der vorliegenden Erfindung zwei Rotorlagerungskonzepte vorgestellt, bei dem das Pumpenlaufrad vollständig berührungslos und somit verschleißfrei im Pumpengehäuse gelagert ist. Diese Konzepte sind:
1) Blutpumpe mit elektromagnetischem Radiallager und permanentmagnetischem Axiallager
2) Blutpumpe mit hydrostatischem Radiallager und hydrodynamischem Axiallager
   Für den kurz- bis mittelfristigen Einsatz, bei dem vorwiegend eine extrakorporale Platzierung der Blutpumpe angemessen ist, wird im Rahmen der vorliegenden Erfindung ein Rotorlagerungs-konzept vorgestellt, welches als Hybridlager auf einer Kombination von mehrerer Lagerprinzipien beruht. Diese ist insbesondere eine:
3) Blutpumpe mit mechanisch-magnetischer Rotorlagerung

Des Weiteren werden im Rahmen der vorliegenden Erfindung, Modifikationen der einzelnen Varianten aufgezeigt, welche sowohl klinische und patientenrelevante Vorteile hervorbringen als auch zur Erhöhung der Betriebssicherheit und zur Senkung des Herstellungsaufwandes beitragen.

Es ist zweckmäßig, die Blutpumpe so auszugestalten, dass sie mindestens zwei räumlich voneinander getrennte Elemente enthält, wobei wenigstens eines der Elemente so beschaffen ist, dass es axiale Rückstellkräfte aufbringt, wobei wenigstens ein weiteres Element eine berührungslose Lagerung bewirkt und wobei das weitere Element ein strömungsmechanisch stabilisierendes Lager ist und wobei die strömungsmechanische Stabilisierung auf einer Wechselwirkung zwischen einer Deckscheibe und dem Pumpengehäuse beruht.

Es ist ferner zweckmäßig, die Blutpumpe so auszugestalten, dass sie mindestens zwei räumlich voneinander getrennte Elemente enthält, wobei wenigstens eines der Elemente so beschaffen ist, dass es eine Deckscheibe aufweist, die so gestaltet ist, dass sie in einer radialen Richtungskomponente strömungsmechanisch stabilisierend wirkt und mindestens ein weiteres Element ein radiales Magnetlager darstellt.

Weitere Vorteile, Besonderheiten und zweckmäßige Weiterbildungen der Erfindung ergeben sich aus den Unteransprüchen und der nachfolgenden Darstellung bevorzugter Ausführungsbeispiele anhand der Abbildungen.

Die Abbildungen zeigen Einzelheiten der erfindungsgemäßen Blutpumpe oder Teile davon in schematischer Form.

Von den Abbildungen zeigt:
- Fig. 1:: Blutpumpe mit elektromagnetischer Rotorlagerung, bei dem als Antrieb ein integrierter Elektromotor und eine permanentmagnetische Axialkupplung verwendet ist
- Fig. 2:: eine Detailansicht zu Fig. 1 mit Darstellung der Strömungsführung
- Fig. 3:: Blutpumpe mit elektromagnetischer Rotorlagerung, bei der als Antrieb ein integrierter Elektromotor mit elektromagnetischer Axialkupplung verwendet ist
- Fig. 4:: Blutpumpe mit elektromagnetischer Rotorlagerung, bei der als Antrieb ein externer Elektromotor mit elektromagnetischer Axialkupplung verwendet ist
- Fig. 5:: Blutpumpe mit elektromagnetischer Rotorlagerung, bei der als Antrieb ein externer Elektromotor mit elektromagnetischer Radialkupplung verwendet ist
- Fig. 6:: eine Detailansicht zu Fig. 5 mit Darstellung der Strömungsführung
- Fig. 7:: Blutpumpe mit elektromagnetischer Rotorlagerung, bei der als Antrieb ein externer Elektromotor mit elektromagnetischer Diagonalkupplung verwendet ist
- Fig. 8:: eine Detailansicht zu Fig. 7 mit Darstellung der strömungsführung
- Fig. 9:: Blutpumpe mit strömungsmechanischer Rotorlagerung, bei der als Antrieb ein integrierter Elektromotor und eine permanentmagnetische Axialkupplung verwendet ist
- Fig. 10:: eine Detailansicht zu Fig. 9 mit Darstellung der Strömungsführung
- Fig. 11:: einen Querschnitt durch das Laufrad aus Fig. 9
- Fig. 12:: einen abgewickelten Querschnitt durch das hydrodynamische Axiallager aus Fig. 9
- Fig. 13:: Blutpumpe mit strömungsmechanischer Rotorlagerung, bei der als Antrieb ein externer Elektromotor mit elektromagnetischer Axialkupplung verwendet ist
- Fig. 14:: Blutpumpe mit strömungsmechanischer Rotorlagerung, bei der als Antrieb ein externer Elektromotor mit elektromagnetischer Diagonalkupplung verwendet ist
- Fig. 15:: Blutpumpe mit mechanisch-magnetischer Rotorlagerung, bei der das mechanische Lager und das magnetische Lager auf gleicher axialer Höhe positioniert sind
- Fig. 16:: eine Detailansicht zu Fig. 15 mit Darstellung der Strömungsführung
- Fig. 17:: Blutpumpe mit mechanisch-magnetischer Rotorlagerung, bei der das mechanische Lager und das magnetische Lager auf ungleicher axialer Höhe positioniert sind
- Fig. 18:: eine Detailansicht zu Fig. 17 mit Darstellung der Strömungsführung
- Fig. 19:: einen Querschnitt durch das Laufrad und das Magnetlager aus Fig. 18
- Fig. 20:: Blutpumpe mit mechanisch-magnetischer Rotorlagerung, bei der das Magnetlager in der Deckscheibe integriert ist
- Fig. 21:: eine Detailansicht zu Fig. 20 mit Darstellung der Strömungsführung
- Fig. 22:: Blutpumpe mit mechanisch-magnetischer Rotorlagerung, bei der das Magnetlager in der Diagonalkupplung implementiert ist
- Fig. 23:: eine Detailansicht zu Fig. 22 mit Darstellung der Strömungsführung
- Fig. 24:: eine Darstellung der Diagonalkupplung zu Fig. 22 ohne Teilung der Kupplungsmagnete
- Fig. 25:: einen Querschnitt durch die Diagonalkupplung aus Fig. 24
- Fig. 26:: eine Darstellung der Diagonalkupplung zu Fig. 24 in ihrer Gleichgewichtslage
- Fig. 27:: eine Darstellung der Diagonalkupplung zu Fig. 24 in ihrer ausgelenkten Position und der dabei wirksamen Lagerkräfte in der Diagonalkupplung
- Fig. 28:: eine Darstellung der Diagonalkupplung zu Fig. 22 mit Teilung der Kupplungsmagnete in radialer Richtung
- Fig. 29:: einen Querschnitt durch die Diagonalkupplung aus Fig. 28
- Fig. 30:: eine Darstellung der Diagonalkupplung zu Fig. 28 in ihrer Gleichgewichtslage
- Fig. 31:: eine Darstellung der Diagonalkupplung zu Fig. 28 in ihrer ausgelenkten Position und der dabei wirksamen Lagerkräfte in der Diagonalkupplung
- Fig. 32:: Blutpumpe mit elliptischem Pumpendesign
- Fig. 33:: eine Schnittdarstellung der Blutpumpe mit elliptischem Pumpendesign
- Fig. 34:: einen Querschnitt durch den elliptischen Motor in 2-phasiger Ausführung
- Fig. 35:: einen Querschnitt durch den elliptischen Motor in 4-phasiger Ausführung
- Fig. 36:: Blutpumpe mit separater Antriebseinheit, wobei der Antrieb des Pumpenkopfes über einen Elektromotor erfolgt
- Fig. 37:: Blutpumpe mit separater Antriebseinheit, wobei der Antrieb des Pumpenkopfes über eine sauerstoffbetriebene Turbine erfolgt
- Fig. 38:: Schematische Darstellung der Blutpumpe aus Fig. 27 beim Patienteneinsatz als reines Blutfördersystem (ohne Oxygenator)
- Fig. 39:: Schematische Darstellung der Blutpumpe aus Fig. 27 beim Patienteneinsatz in Kombination mit einem Blutoxygenationssystem (Oxygenator)
- Fig. 40:: Schematische Darstellung einer implantierbaren Blutpumpe als VAD-System
- Fig. 41:: Schematische Darstellung einer extrakorporalen Blutpumpe im ECMO-System

Die Abbildungen Fig. 1 bis Fig. 8 beziehen sich auf eine Blutpumpe mit elektromagnetischer Radiallagerung und permanentmagnetischer Axiallagerung.

Die Abbildungen Fig. 9 bis Fig. 14 beziehen sich auf eine Blutpumpe gemäß der Erfindung mit hydrostatischer Radiallagerung und hydrodynamischer Axiallagerung

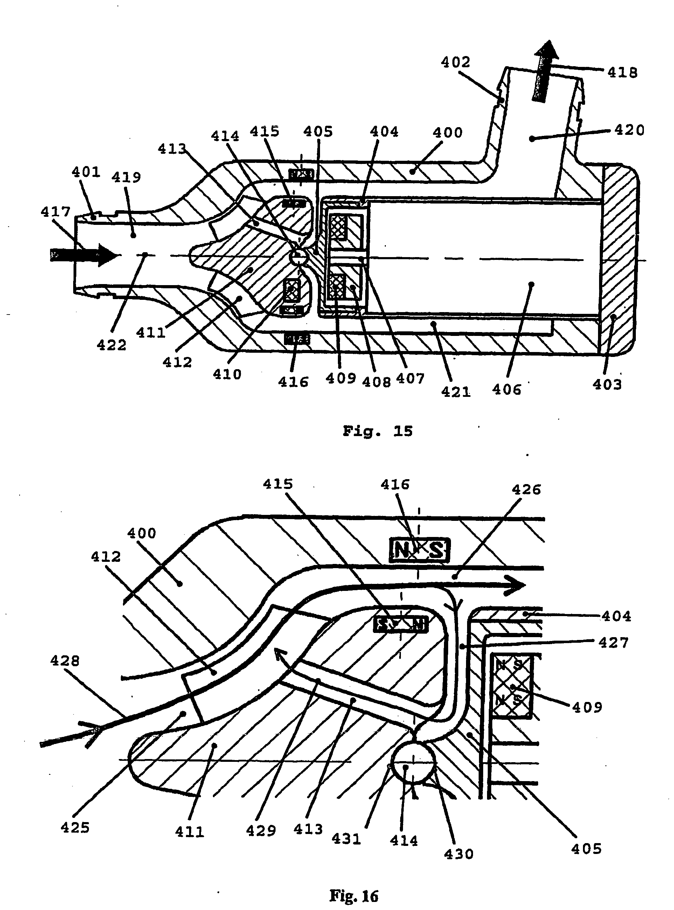

Die Abbildungen Fig. 15 bis Fig. 31 beziehen sich auf eine Blutpumpe mit mechanisch-magnetischer Rotorlagerung.

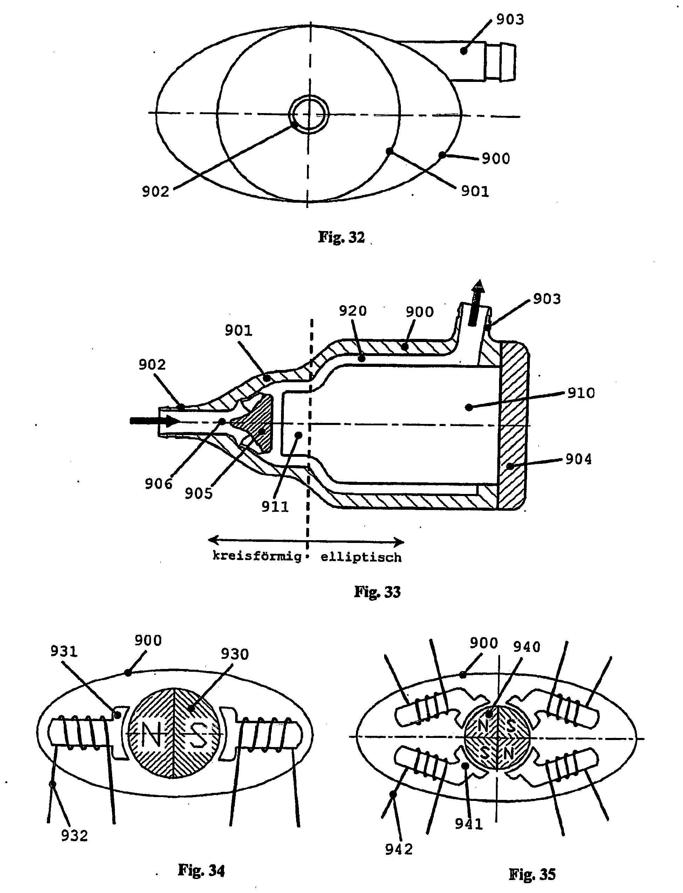

Die Abbildungen Fig. 32 bis Fig. 35 beziehen sich auf eine Blutpumpe mit harmonischer Flachbauweise der Blutpumpe.

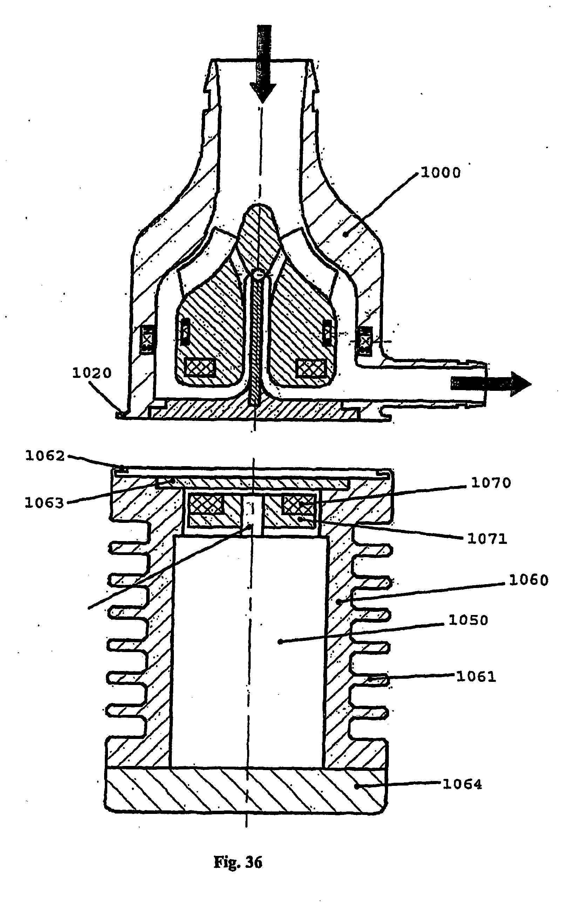

Die Abbildung Fig. 36 bezieht sich auf eine Blutpumpe gemäß einer der vorangehenden Abbildungen (insbesondere mechanischmagnetische Rotorlagerung), wobei die Blutpumpe als trennbarer Pumpenkopf von einem externen Elektromotor angetrieben wird.

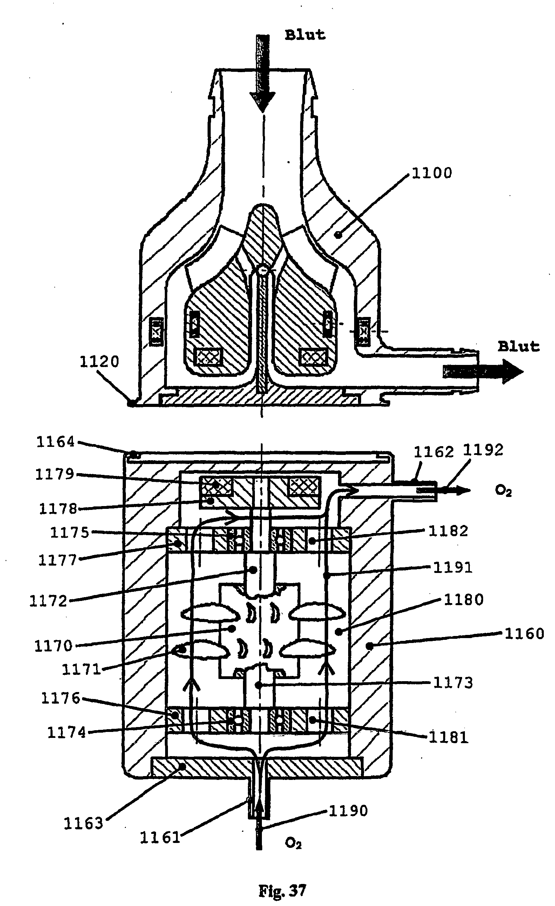

Die Abbildungen Fig. 37 bis Fig. 39 beziehen sich auf eine Blutpumpe wobei die Blutpumpe als trennbarer Pumpenkopf über eine externe Turbine angetrieben wird.

Die Abbildungen Fig. 40 bis Fig. 41 beziehen sich auf den allgemeinen Stand der Technik.

Die Abbildungen Fig. 1 bis Fig. 35 beziehen sich insbesondere auf eine langfristige Anwendung (VAD, ECMO, etc.), bei der die Blutpumpe implantierbar gestaltet ist, wie dies beispielhaft in Fig. 40 illustriert ist.

Die Abbildungen Fig. 36 bis Fig. 39 beziehen sich insbesondere auf eine kurz- bis mittelfristige Anwendungen (HLM, ECMO, Kurzzeit-VAD, etc.), bei der die Blutpumpe extrakorporal platziert wird.

Figur 1 und Figur 2 zeigen eine Blutpumpe, bei der die Lagerung des Pumpenlaufrades (12) im Pumpengehäuse (1) in radialer Richtung über ein elektromagnetisches Lager (19, 20) und in axialer Richtung über ein permanentmagnetisches Lager (16, 17) erfolgt. Der Antrieb des Laufrades (12) erfolgt hier über einen Elektromotor (5), welcher im Pumpengehäuse (1) integriert ist, wobei die Übertragung der Drehbewegungen vom Motor (5) auf das Laufrad (12) über eine permanentmagnetische Axialkupplung (8, 9, 11) erfolgt. Der Elektromotor (5) befindet sich in einem Motorgehäuse (7) und ist weiterhin über den Motordeckel (10) hermetisch gegen den blutdurchströmten Raum (15) abgedichtet.

Die permanentmagnetische Kupplung (8, 9) ist antriebsseitig über den Polschuh (8) drehstarr mit der Motorwelle (6) verbunden. Der Polschuh (8) gewährleistet hierbei die magnetische Rückkopplung der einzelnen Antriebsmagnete (9) und ist aus einem magnetisch leitenden Werkstoff (z.B. magnetisierbarer Stahl) gefertigt. Die Kupplungsmagnete (9, 11) stellen Dauermagnete dar, die insbesondere aus hochleistungsfähigen Magnetwerkstoffen (Selten-Erdmagnete) wie z.B. Neodym-Eisen-Bor (NdFeB) oder Samarium-Cobalt (SmCo) gefertigt sind. Abtriebsseitig sind die Kupplungsmagnete (11) im Pumpenlaufrad (12) eingebettet und haben somit keinen Kontakt mit dem Fördermedium (Blut), sodass Korrosionsgefahren der Kupplungsmagnete (11) im Blutstrom ausgeschlossen werden können. Die beiden Kupplungshälften (9, 11) bestehen jeweils aus einer geraden Anzahl (bevorzugt 2, 4, 6 oder 8) von einzelnen Magnetsegmenten, die zusammengesetzt einen Vollkreis ergeben. Als Illustration hierfür kann die Abbildung Fig. 11 zugrunde gelegt werden. Die einzelnen Magnetsegmente (11 bzw. 238) sind hierbei wechselweise in axialer Richtung polarisiert. Auf der Antriebsseite der Magnetkupplung (9) befindet sich ebenfalls eine gleiche Anzahl von Magnetsegmenten, die ebenfalls wechselweise in axialer Richtung polarisiert sind. Die Antriebsmagnete (9) stehen den Abtriebsmagneten (11) nun derart gegenüber, dass zwischen ihnen magnetische Anziehungskräfte in axialer Richtung wirken, wie dies in Fig. 2 zu erkennen ist. Bei Drehbewegungen der Motorwelle (6) und damit der antriebsseitigen Kupplungshälfte (8, 9) werden die beiden Kupplungshälften gegeneinander soweit verdreht, bis das Luftspaltmoment in der Magnetkupplung (9, 11) dem Lastmoment auf der Laufradseite (12) entspricht und somit die Drehbewegungen vom Motor (5) auf das Pumpenlaufrad (12) übertragen werden. Das Rückstellmoment in der Magnetkupplung (9, 11) ist derart ausgelegt, dass die für den Betrieb der Blutpumpe erforderlichen Lastmomente ohne Entkopplung übertragen werden können. Dies wird insbesondere durch die wechselweise Polarisierung der einzelnen Magnetsegmente erreicht und kann bei Erhöhung der Polzahl der Kupplung entsprechend gesteigert werden.

Der Elektromotor (5) stellt hierbei einen kommerziellen Elektromotor mit ausreichender Lebensdauer (mindestens 6 Monate) und ausreichender Leistungsfähigkeit (ca. 20 Watt) dar, sodass der Antrieb des Pumpenlaufrades (12) über den gesamten Betriebsbereich (0 bis 7 L/min, 0 bis 200 mmHg, 0 bis 10.000 min-1) der Blutpumpe gewährleistet ist.

Das Pumpenlaufrad (12) führt somit im Betrieb der Blutpumpe Drehbewegungen um ihre Drehachse (23) aus und muss hierbei in axialer Richtung mit Hilfe einer geeigneten Rotorlagerung stabilisiert werden, sodass ein Anschlagen des Pumpenlaufrades (12) am Pumpengehäuse (1) vermieden wird. In axialer Richtung wirken auf das Pumpenlaufrad (12) neben den magnetischen Anziehungskräften der Magnetkupplung (9, 11) auch die entgegengerichteten hydraulischen Strömungskräfte (Axialschub).

In Fig. 1 und Fig. 2 wird die axiale Lagerung des Pumpenlaufrades (12) im Pumpengehäuse (1) mit Hilfe eines permanentmagnetischen Axiallagers (16, 17) erreicht. Das permanentmagnetische Axiallager besteht hierbei aus einer dauermagnetischen Vorrichtung (17) (Statormagnet), welche im Pumpengehäuse (1) unbeweglich positioniert ist sowie einer weiteren dauermagnetischen Vorrichtung (16) (Rotormagnet), welches im Pumpenlaufrad (12) oder in einem ihrer Teilkomponenten wie z.B. der Deckscheibe (18) oder den Laufradschaufeln (13) integriert ist und somit die gleichen Drehbewegungen um die Drehachse (23) ausführt wie das Pumpenlaufrad selbst. Eine Möglichkeit der Ausführung des permanentmagnetischen Axiallagers ist in Fig. 1 und Fig. 2 dargestellt. Hierbei bestehen der Statormagnet (17) und der Rotormagnet (16) jeweils aus dauermagnetischen Ringen, die in axialer Richtung entgegengerichtet polarisiert sind. Der Rotormagnet (16) ist hierbei in der Deckscheibe (18) integriert und steht dem Statormagneten (17) im Pumpengehäuse radial gegenüber. Der radiale Luftspalt zwischen Statormagnet (17) und Rotormagnet (16) beträgt in der bevorzugten Ausführung der Blutpumpe zwischen 0,1 und 5,0 mm, insbesondere zwischen 0,5 und 2,0 mm.

Bei axialen Auslenkungen des Pumpenlaufrades (12) im Pumpengehäuse (1) werden zwischen dem Statormagnet (17) und dem Rotormagnet (16) dauermagnetische Kräfte wirksam, welche eine axiale Rückstellung des Pumpenlaufrades (12) im Pumpengehäuse (1) bewirken. Die axiale Auslenkung des Pumpenlaufrades (12) im Pumpengehäuse (1) wird mit Hilfe eines solchen Axiallagers derart eingegrenzt, dass über den gesamten Betriebsbereich der Blutpumpe eine axiale Berührung des Pumpenlaufrades (12) und ihrer einzelnen Teilkomponenten wie Deckscheibe (18) und Laufradschaufeln (13) mit dem Pumpengehäuse (1) oder mit anderen stationären Pumpenelementen (10) vermieden wird. Dies wird bei der vorliegenden Erfindung durch den Einsatz von Hochleistungsmagneten (Seltenerdmagneten) wie z.B. NdFeB oder SmCo als Werkstoffe für die permanentmagnetische Axiallagerung (16, 17) erreicht, deren magnetische Rückstellkraft bei axialen Auslenkungen des Pumpenlaufrades (12) im Pumpengehäuse (1) in axialer Richtung betragsmäßig größer ist als die in der Magnetkupplung (9, 11) wirkende axiale Anziehungskraft. Da das permanentmagnetische Axiallager (16, 17) in axialer Richtung wechselseitig belastet werden kann, ist somit auch sichergestellt, dass die der Kupplungskraft entgegengerichtete hydraulische Strömungskraft (Axialschub) durch das Magnetlager (16, 17) aufgefangen wird. Die hierbei auftretenden axialen Auslenkungen des Pumpenlaufrades (12) im Betrieb der Pumpe liegen zwischen 0 und 5,0 mm, insbesondere zwischen 0,01 und 1,0 mm.

Neben den axialen Rückstellkräften wirken im permanentmagnetischen Axiallager (16, 17) gleichzeitig auch instabile radiale Anziehungskräfte, die mit zunehmender radialer Auslenkung des Pumpenlaufrades (12) im Pumpengehäuse (1) stetig zunehmen und durch ein weiteres Radiallager stabilisiert werden müssen.

Die radiale Lagerung des Pumpenlaufrades (12) im Pumpengehäuse (1) wird bei der vorliegenden Erfindung durch ein zusätzliches elektromagnetisches Lager (19, 20) ermöglicht, welches räumlich vom elektromagnetischen Antrieb (5, 6, 8, 9) getrennt ist. Eine derartige Trennung des elektromagnetischen Lagers (19, 20) vom elektromagnetischen Antrieb (5, 6, 8, 9) bringt bei der vorliegenden Erfindung den wesentlichen Vorteil, dass bei Störungen und Ausfällen des elektromagnetischen Lagers (19, 20) der Antrieb des Pumpenlaufrades (12) erhalten bleibt und die Blutpumpe somit bei Notlaufeigenschaften weiterbetrieben werden kann ohne dass der Patient eine hämodynamische Einbuße erleidet. Weiterhin bietet eine derartige Ausführung von elektromagnetischer Rotorlagerung und elektromagnetischem Antrieb den Vorteil, dass die Antriebseinheit und damit die gesamte Blutpumpe wesentlich kompakter in ihrer Baugröße gestaltet werden kann, wodurch die Implantation der Blutpumpe weiter begünstigt wird.

Das elektromagnetische Radiallager (19, 20) besteht aus einer Statorvorrichtung (19) sowie einer Rotorvorrichtung (20), zwischen denen bei radialen Auslenkungen des Pumpenlaufrades (12) im Pumpengehäuse (1) elektromagnetische Kräfte in radialer Richtung wirksam werden, derart, dass eine radiale Berührung des Pumpenlaufrades (12) und ihrer einzelnen Komponenten (13, 18) mit dem Pumpengehäuse (1) vermieden wird. Dies wird bei der vorliegenden Erfindung durch eine elektromagnetische Statorvorrichtung (19) sowie eine weitere dauermagnetische Rotorvorrichtung (20) ermöglicht. Die magnetische Rotorvorrichtung (20) ist im Pumpenlaufrad (12) oder in einem ihrer Teilkomponenten wie Deckscheibe (18) oder Laufradschaufeln (13) integriert und führt somit die gleichen Drehbewegungen um die Drehachse (23) aus wie das Pumpenlaufrad (12) selbst. Eine Möglichkeit der Ausführung des elektromagnetischen Radiallagers (19, 20) ist durch den Einsatz von Dauermagneten oder magnetisierbaren Werkstoffen als Rotorvorrichtung (20) gegeben. Die Statorvorrichtung (20) hingegen besteht aus geregelten Elektromagneten, welche in einer bestimmten Anzahl (bevorzugt 1 bis 5, insbesondere 2 bis 3) im Pumpengehäuse (1) integriert und in Umfangsrichtung verteilt sind. Der radiale Abstand zwischen dem Pumpenlaufrad (12) und dem Pumpengehäuse (1) bzw. zwischen der Rotorvorrichtung (20) und dem Pumpengehäuse (1) wird hierbei über Wegmesssensoren (z.B. induktive Sensoren, kapazitive Sensoren, optische Sensoren oder Ultraschallsensoren) erfasst, die in der Statorvorrichtung (19) oder im Pumpengehäuse (1) implementiert sind. Bei radialen Auslenkungen des Laufrades (12) im Pumpengehäuse (1) werden über das gemessene Signal die Elektromagneten in der Statorvorrichtung (19) derart aktiviert, dass zwischen der Statorvorrichtung (19) und der Rotorvorrichtung (20) elektromagnetische Kräfte in radialer Richtung wirksam werden, die eine Rückstellung des Pumpenlaufrades (12) in die konzentrische Lage im Pumpengehäuse (1) hervorrufen.

Es besteht eine Beschriebene Blutpumpe auch darin, dass das permanentmagnetische Axiallager (16, 17) und das elektromagnetische Radiallager (19, 20) in einer Einheit vereint sind, die in ihrer Gesamtheit vom elektromagnetischen Antrieb (5, 6, 8, 9) räumlich getrennt bleibt und somit die oben genannten Vorteile einer räumlichen Trennung von Rotorlagerung und Pumpenantrieb weiter aufrechterhält. Eine Vereinigung der elektromagnetischen Radiallagerung mit der permanentmagnetischen Axiallagerung bringt darüber hinaus den Vorteil, dass unter Umständen die gesamte Rotorlagerung und damit auch die Blutpumpe in ihrer Baugröße kompakter und damit implantationsfreundlicher gestaltet werden kann.

Für die Notlaufeigenschaften der Blutpumpe mit einer derartigen Rotorlagerung sind im bestimmte Vorkehrungen getroffen, die im Folgenden näher erläutert werden sollen. Da die Rotorlagerung in axialer Richtung über ein permanentmagnetisches Axiallager (16, 17) verfügt, bedarf es aufgrund der Wartungsfreiheit und der unbegrenzten Lebensdauer der Dauermagneten keiner weiteren Sicherheitsvorkehrung zur Aufrechterhaltung der axialen Rotorstabilität. Für die Lagerung des Pumpenlaufrades (12) in radialer Richtung hingegen müssen Vorkehrungen getroffen werden, welche eine Stabilisierung des Laufrades (12) im Pumpengehäuse (1) auch bei Störungen oder Ausfall des elektromagnetischen Radiallagers aufrechterhalten.

Hierzu ist ein strömungsmechanisches Radiallager vorgesehen, dessen Funktionsweise aus Fig. 2 zu ersehen ist. Der radiale Spalt zwischen der Deckscheibe (18) und dem gegenüberliegenden Pumpengehäuse (1) bringt einen Strömungsraum (32) hervor, welcher infolge der zuvor beschriebenen Druckverteilung am Laufrad (12) in axialer Richtung retrograd zum Hauptstrom (31) von einer Leckageströmung (35) durchströmt wird. Ursache für diese vorwiegend in axialer Richtung verlaufende Leckageströmung (35) ist wie bereits erwähnt der hydrostatische Druckunterschied zwischen der Einmündung (37) und der Ausmündung (38) des Spaltraumes (32). Bei konzentrischer Lage des Pumpenlaufrades (12) im Pumpengehäuse (1) liegt im Spaltraum (32) eine in Umfangsrichtung konstante Druckverteilung vor, sodass auch keine radialen Kräfte auf die Deckscheibe (18) bzw. das Pumpenlaufrad (12) einwirken. Bei radialen Auslenkungen des Pumpenlaufrades (12) im Pumpengehäuse (1) hingegen wird die Druckverteilung im nunmehr exzentrischen Spaltraum (32) in Umfangsrichtung derart verändert, dass der hydrostatische Druck im engeren Spaltbereich größer wird als der hydrostatische Druck im diametral gegenüberliegenden vergrößerten Spaltbereich, sodass daraus eine radiale Rückstellkraft auf die Deckscheibe (18) und damit auch auf das Pumpenlaufrad (12) resultiert, welche das Laufrad wieder in die konzentrische Lage im Pumpengehäuse versetzt ("Lomakin-Effekt").

Weiterhin wird infolge der Drehbewegung des Laufrades (12) eine Rotationsströmung im Spaltraum (32) hervorgerufen, welche bei radialen Auslenkungen des Pumpenlaufrades (12) im Pumpengehäuse (1) eine hydrodynamische Radiallagerung hervorbringt ("Reynolds-Effekt").

Auf diese Weise ist sichergestellt, dass auch bei Ausfall des elektromagnetischen Radiallagers (19, 20) das Pumpenlaufrad (12) weiterhin berührungslos im Pumpengehäuse (1) gelagert ist. Der bei Ausfall des elektromagnetischen Radiallagers (19, 20) resultierende Einsatz des strömungsmechanischen Radiallagers ist, aufgrund der weiterhin bestehenden radialen Anziehungskräfte des permanentmagnetischen Axiallagers (16, 17), durch ein Betriebverhalten gekennzeichnet, bei dem infolge der exzentrischen Lage des Pumpenlaufrades (12) im Pumpengehäuse (1) ein örtlich verengter Spaltraum (32) zwischen Deckscheibe (18) und Pumpengehäuse (1) vorliegt. Die daraus resultierende erhöhte Scherbelastung der Blutströmung sowie die damit verbundene Hämolyse wird über den Zeitraum des Notlaufbetriebes in Kauf genommen.

Figur 3 zeigt eine Blutpumpe, bei der die Rotorlagerung und Strömungsführung innerhalb der Blutpumpe den gleichen Aufbau und die gleiche Funktionsweise aufweisen wie die Blutpumpe aus Fig. 1 und Fig. 2. Bei Fig. 3 ist dennoch eine Ausführung der Blutpumpe gezeigt, bei der der integrierte Antrieb (75, 76, 77, 78) nicht über eine permanentmagnetische Kupplung, sondern über eine elektromagnetische Axialkupplung (75, 76) angetrieben wird. Die Vorteile einer derartigen Ausführung liegen einerseits in der Verringerung der Bauteile und damit der Reduzierung der Komplexität der Blutpumpe und andererseits in der höheren Lebensdauer des Antriebes gemäß Fig. 3, da hier der Antrieb des Pumpenlaufrades (73) nicht über einen zusätzlichen Elektromotor (mit Lebensdauerbegrenzung aufgrund der darin verwendeten Wälzlager) erfolgt, sondern direkt über eine elektromagnetische Kupplung (75, 76) gewährleistet ist. Eine elektromagnetische Kupplung basiert gemäß dem allgemeinen Stand der Technik auf einem elektromagnetischen Drehfeld, welches von den Elektromagneten (75, 76) auf die abtriebsseitigen Kupplungsmagnete (74) im Pumpenlaufrad (73) berührungslos übertragen wird. Die weitere Funktionsweise der Blutpumpe insbesondere der permanentmagnetischen Axiallagerung (80, 81) sowie der elektromagnetischen Radiallagerung (82, 83) des Pumpenlaufrades (73) im Pumpengehäuse (70) ist analog zu der Ausführung der Blutpumpe aus Fig. 1 und Fig. 2.

Figur 4 zeigt eine Blutpumpe bei der der gleiche Aufbau und die gleiche Funktionsweise der Blutpumpe vorliegt wie in Fig. 3. Bei Fig. 4 liegt dennoch eine Ausführung der Blutpumpe vor, bei der der elektromagnetische Antrieb (107, 108, 109) nicht im Innenraum des Pumpengehäuses (100) integriert ist, sondern außerhalb dieser an der Rückseite des Pumpengehäuses (114) befestigt ist. Die Vorteile einer derartigen Ausführung liegen einerseits in der kompakteren Bauweise des Pumpengehäuses (100) und damit unter Umständen auch der gesamten Blutpumpe, wodurch sowohl die Implantierbarkeit verbessert wird als auch der Kontakt der Blutströmung mit Fremdoberflächen minimiert werden kann. Da die axialen Abmessungen des Pumpengehäuses (100) weitgehend reduziert sind, bietet sich bei dieser Ausführung zusätzlich die Möglichkeit an, den Pumpenauslass (102) näher an den Laufradbereich (115, 116) zu positionieren und bei der Gestaltung des Pumpenlaufrades (103) neben der diagonalen Laufradform auch die radiale Laufradform zu verwenden. Die Vorteile einer radialen Laufradform im Vergleich zu einer diagonalen Laufradform, wie dies bei den Ausführungen in Fig. 1 und Fig. 3 wieder zu finden ist, liegen darin, dass bei radialen Laufrädern die Laufraddrehzahl im Betrieb der Blutpumpe infolge der stärker ausgeprägten Zentrifugaleffekte niedriger ist als die der diagonalen Laufradform. Eine Reduzierung der Laufraddrehzahl bringt neben den geringeren Belastungen der einzelnen Komponenten der Blutpumpe insbesondere den Vorteil, das die Scherbelastungen des Blutes und damit die Hämolyserate reduziert werden kann.

Die Wirkungsweise der elektromagnetischen Axialkupplung (106, 107, 108) entspricht der Kupplungsvorrichtung aus Fig. 3.

Die weitere Funktionsweise der Blutpumpe insbesondere der permanentmagnetischen Axiallagerung (110, 111) sowie der elektromagnetischen Radiallagerung (112, 113) des Pumpenlaufrades (103) im Pumpengehäuse (100) ist analog zu der Ausführung der Blutpumpe aus Fig. 1 und Fig. 3.

Figur 5 und Figur 6 zeigen eine Blutpumpe, bei der der Antrieb des Pumpenlaufrades (133) über eine radial wirkende elektromagnetische Kupplung (136, 137, 138, 140) gewährleistet wird. Die Lagerung des Pumpenlaufrades (133) im Pumpengehäuse (130) erfolgt in radialer Richtung analog zu den Ausführungen der Blutpumpe aus Fig. 1, Fig. 3 und Fig. 4 über ein elektromagnetisches Lager (142, 143). Die Lagerung des Laufrades (133) in axialer Richtung hingegen erfolgt bei der Ausführung gemäß Fig. 5 und Fig. 6 über ein magnetisches Axiallager, welches bereits in der Radialkupplung (136, 140) implementiert ist. Eine radial wirkende Magnetkupplung (136, 140) wird bei axialen Auslenkungen der Laufradmagnete (136) gegenüber den Antriebsmagneten (140) eine axiale Rückstellkraft erfahren, welche bestrebt ist, die Antriebmagnete (136) und damit auch das Pumpenlaufrad (133) wieder in die axiale Gleichgewichtslage zu versetzen. Die radial wirkende Magnetkupplung stellt demnach ein magnetisches Axiallager zur Verfügung.

Der wesentliche Vorteil einer derartigen Blutpumpe mit radial wirkender Magnetkupplung liegt demnach darin, dass kein weiteres Axiallager zur Stabilisierung des Pumpenlaufrades (133) im Pumpengehäuse benötigt wird, sodass die Blutpumpe in ihrer Gesamtheit sowohl kompakter als auch leichter herstellbar wird. Bezüglich der Platzierung der Antriebseinheit (137, 138, 139, 140) gelten die gleichen Vorteile wie bei der Ausführung der Blutpumpe gemäß Fig. 4. Bezüglich der räumlichen Trennung des elektromagnetischen Lagers (142, 143) vom elektromagnetischen Antrieb (137, 138, 139, 140) gelten die gleichen Vorteile wie bei der Ausführung der Blutpumpe gemäß Fig. 1, Fig. 3 und Fig. 4. Bezüglich der strömungsmechanischen Rotorlagerung im Strömungsspalt (157) zwischen der Deckscheibe (150) und dem gegenüberliegenden Pumpengehäuse (130) gelten bei Ausfall des elektromagnetischen Lagers (142, 143) die gleichen Erläuterungen und Vorteile wie bei der Ausführung der Blutpumpe gemäß Fig. 1.

In Bezug auf die Strömungsführung innerhalb der Blutpumpe liegen bei der Ausführung gemäß Fig. 5 dennoch weitere Besonderheiten vor, die anhand von Fig. 6 näher erläutert werden sollen. Aufgrund der radialen Wirkrichtung der Magnetkupplung (136, 140) entsteht auf der Rückseite des Laufrades (133) eine veränderte Strömungsführung, die im Hinblick auf die radiale Stabilisierung des Pumpenlaufrades (133) bei Ausfall des elektromagnetischen Radiallagers (142, 143) und dem daraus resultierenden Notlaufbetrieb der Blutpumpe von zentraler Bedeutung ist.

Die Spalträume (152, 153, 154) zwischen der Rückseite des Pumpenlaufrades (133) und den gegenüberliegenden Bereichen des Pumpengehäuses (141, 144, 146) bilden einen Strömungsraum, welcher infolge der zuvor beschriebenen Druckverteilung am Laufrad (133) retrograd zur Hauptströmung (157) vom Hochdruckgebiet am Laufradaustritt (145) über die einzelnen Spalträume (152, 153, 154) sowie den im Laufradkörper (133) eingearbeiteten Spülkanälen (156) zur Vorderseite des Laufrades (133) durchströmt wird. Dieser Spülstrom (160, 155) gewährleistet einerseits, dass Strömungstagnationen in den einzelnen Strömungsbereichen (152, 153, 154, 155, 156) vermieden werden und somit die Thrombengefahr in der Blutpumpe reduziert wird. Andererseits bietet insbesondere die Durchströmung des Spaltraumes (153) eine bei Ausfall des elektromagnetischen Radiallagers (142, 143) eingreifende strömungsmechanische Rotorlagerung, die bei radialen Auslenkungen des Pumpenlaufrades (133) im Pumpengehäuse (130) das Laufrad (133) in radialer Richtung derart stabilisiert, wie dies bereits bei der strömungsmechanischen Rotorlagerung aus Fig. 1 bzw.

Fig. 2 erläutert wurde. Durch eine Ausführung der Blutpumpe gemäß Fig. 5 und Fig. 6 wird somit ein zusätzliches Notlauflager (153, 160) bereitgestellt, welches in der Gesamtheit zur Erhöhung der Betriebssicherheit der Blutpumpe beiträgt.

Die weitere Funktionsweise der Blutpumpe insbesondere die der elektromagnetischen Radiallagerung (142, 143) des Pumpenlaufrades (133) im Pumpengehäuse (130) ist analog zu der Ausführung der Blutpumpe aus Fig. 1, Fig. 3 und Fig. 4.

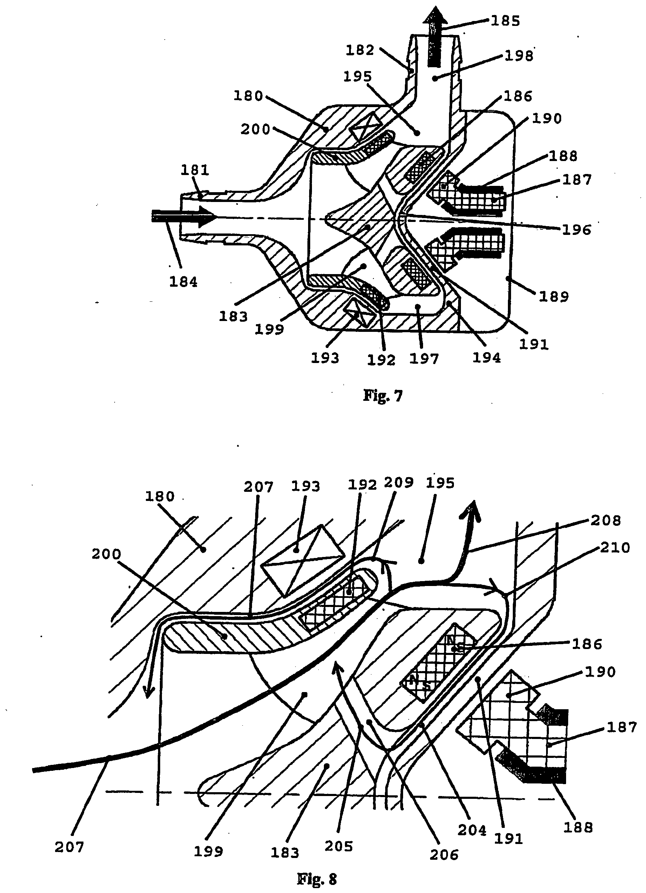

Figur 7 und Figur 8 zeigen eine Blutpumpe bei der der Antrieb des Pumpenlaufrades (183) über eine diagonal wirkende elektromagnetische Kupplung (186, 187, 188, 189, 190) gewährleistet wird. Die Lagerung des Pumpenlaufrades (183) im Pumpengehäuse (180) erfolgt hierbei ebenfalls in diagonaler Richtung und kombiniert im Gegensatz zu den vorangehenden Ausführungen der Blutpumpe die Radiallagerung und Axiallagerung des Laufrades (183) in einer Einheit, wobei die elektromagnetischen Rückstellkräfte in der Magnetlagerung (192, 193) je nach Auslenkung des Laufrades (183) im Pumpengehäuse (180) eine entsprechende Komponente in axialer Richtung und eine weitere Komponente in radialer Richtung aufweisen. Da die Wirkrichtung der elektromagnetischen Statorvorrichtung (193) in diagonaler Richtung verläuft, wird die Ausrichtung der Rotorvorrichtung (192) in einer bevorzugten Ausführung der Blutpumpe ebenfalls in diagonaler Richtung wirksam werden. Da die elektromagnetischen Anziehungskräfte in der Magnetkupplung (186, 190) ebenfalls in einer diagonalen Richtung verlaufen, werden diese Anziehungskräfte bei ausreichend kleinen Betriebstoleranzen bereits eine radiale Zentrierung des Laufrades (183) im Pumpengehäuse (180) bewirken, sodass der elektromagnetischen Rotorlagerung (192, 193) vornehmlich die Aufgabe eines Axiallagers zukommt. Lediglich bei unerwarteten externen Stoßkräften (z.B. Sturz des Patienten) wird die Magnetlagerung (192, 193) ebenfalls in radialer Richtung aktiv werden.

Der wesentliche Vorteil einer derartigen Ausführung liegt in der kompakteren Ausführung der Rotorlagerung und damit verbunden auch in der Kompaktheit der gesamten Blutpumpe, was bei der Implantation entsprechende Vorteile für den chirurgischen Eingriff mit sich bringt.

Bezüglich der Platzierung des Antriebseinheit (187, 188, 189, 190) gelten die gleichen Vorteile wie bei der Ausführung der Blutpumpe gemäß Fig. 4 und Fig. 5. Bezüglich der räumlichen Trennung des elektromagnetischen Lagers (192, 193) vom elektromagnetischen Antrieb (187, 188, 189, 190) gelten die gleichen Vorteile wie bei der Ausführung der Blutpumpe gemäß Fig. 1, Fig. 3, Fig. 4 und Fig. 5. Bezüglich der strömungsmechanischen Rotorlagerung im Strömungsspalt (207) zwischen der Deckscheibe (200) und dem gegenüberliegenden Pumpengehäuse (180) bei Ausfall des elektromagnetischen Lagers (192, 193) gelten die gleichen Erläuterungen und Vorteile wie bei der Ausführung der Blutpumpe gemäß Fig. 1.

In Bezug auf die Strömungsführung innerhalb der Blutpumpe liegen bei der Ausführung gemäß Fig. 7 dennoch weitere Besonderheiten vor, die anhand von Fig. 8 näher erläutert werden sollen. Aufgrund der diagonalen Wirkrichtung der Magnetkupplung (186, 190) entsteht auf der Rückseite des Laufrades (183) eine veränderte Strömungsführung, die im Hinblick auf die radiale Stabilisierung des Pumpenlaufrades (183) bei Ausfall des elektromagnetischen Radiallagers (192, 193) und dem daraus resultierenden Notlaufbetrieb der Blutpumpe von zentraler Bedeutung ist.

Der Spaltraum (204) zwischen der Rückseite des Pumpenlaufrades (183) und dem gegenüberliegenden Pumpengehäuse (191) bildet einen Strömungsraum, welcher infolge der zuvor beschriebenen Druckverteilung am Laufrad (183) retrograd zur Hauptströmung (207) vom Hochdruckgebiet am Laufradaustritt (195) über den Spaltraum (204) sowie den im Laufradkörper (183) eingearbeiteten Spülkanälen (206) zur Vorderseite des Laufrades (183) durchströmt wird. Dieser Spülstrom (210, 205) gewährleistet einerseits, dass Strömungstagnationen in den einzelnen Strömungsbereichen (204, 206) vermieden werden und somit die Thrombengefahr in der Blutpumpe herabgesetzt wird. Andererseits bietet insbesondere die Durchströmung des Spaltraumes (204) eine bei Ausfall des elektromagnetischen Lagers (192, 193) eingreifende strömungsmechanische Rotorlagerung, die bei radialen Auslenkungen des Pumpenlaufrades (183) im Pumpengehäuse (180) das Laufrad (183) in radialer Richtung stabilisiert, wie dies bereits bei der strömungsmechanischen Rotorlagerung aus Fig. 1 bzw. Fig. 2 erläutert wurde.

Durch eine Ausführung der Blutpumpe gemäß Fig. 7 und Fig. 8 wird somit ein zusätzliches Notlauflager (204, 210) bereitgestellt, welches in der Gesamtheit zur Erhöhung der Betriebssicherheit der Blutpumpe beiträgt. Weiterhin weist die Umspülung des Spaltraumes (204) eine besonders blutschonende Strömungsführung auf, da hier die Spülströmung (210, 205) nur einem sehr geringen Maß an Strömungsumlenkungen ausgesetzt wird und somit die Gefahr der Strömungsablösung und der daraus resultierenden Thrombengefahr in rezirkulierenden Strömungsbereichen weitgehend unterbunden wird.

Die weitere Funktionsweise der Blutpumpe entspricht weitgehend der Ausführung der Blutpumpe aus Fig. 1, Fig. 3, Fig. 4 und Fig. 5.

Figur 9, Figur 10, Figur 11 und Figur 12 zeigen eine Blutpumpe, bei der die Lagerung des Pumpenlaufrades (234) im Pumpengehäuse (230) in radialer Richtung über ein hydrostatisches Lager (247) und in axialer Richtung über ein hydrodynamisches Lager (239) erfolgt. Der Antrieb des Laufrades (12) erfolgt hier über einen Elektromotor (233) und eine permanentmagnetische Axialkupplung (235, 236, 237, 238), welche in ihrem Aufbau und ihrer Funktionsweise der Ausführung der Blutpumpe aus Fig. 1 und Fig. 2 entspricht. Die zuvor erwähnten Vorteile eines derartigen Antriebskonzeptes gelten daher ebenfalls für die Ausführung der Blutpumpe gemäß Fig. 9 und Fig. 10.

Bezüglich der Strömungsführung innerhalb der Blutpumpe gelten die im Abschnitt "Aufbau und Funktionsweise der Blutpumpe" angeführten Beschreibungen und Erläuterungen. Darüber hinaus kommen bei der Blutpumpe gemäß der Ausführung aus Fig. 9 und Fig. 10 den Sekundärströmungen (252, 253) eine zentrale Rolle bezüglich der Wirkungsweise der hydrostatischen und der hydrodynamischen Rotorlagerung zu, die im Folgenden näher erläutert werden sollen.

Infolge der axialen Anziehungskräfte zwischen den Antriebsmagneten (237) und den Abtriebsmagneten (238) der permanentmagnetischen Axialkupplung (235, 236, 237, 238) wirkt auf das Pumpenlaufrad (234) eine Axialkraft, welche zur Folge hat, dass das Laufrad (234) in Richtung der Antriebseinheit bewegt wird, bis es an ihrer Rückseite (239) mit dem stationären Pumpenelement (240) in Berührung kommt. Zur Lagerung des Laufrades (234) im Pumpengehäuse (230) bedarf es daher einer Axiallagerung, welche den axialen Anziehungskräften der Magnetkupplung (235, 236, 237, 238) entgegenwirkt und die axialen Bewegungen des Laufrades (234) eingrenzt. Bei der Ausführung der Blutpumpe gemäß Fig. 9 und Fig. 10 wird dies über ein hydrodynamisches Axiallager auf der Laufradrückseite (239) erreicht, deren Aufbau und Funktionsweise im Folgenden anhand von Fig. 10 näher beschrieben wird.

Die Umspülung der Laufradrückseite (259) erfolgt bei der Ausführung der Blutpumpe gemäß Fig. 9, Fig. 10, Fig. 11 und Fig. 12 über einen Spülstrom (251, 252), der auf der Laufradvorderseite (254) vom Hauptstrom (250) abgezweigt wird und über einen zentralen Spülkanal (248) im Laufradkörper (234) auf die Laufradrückseite (259) weitergeleitet wird. Die auf der Rückseite des Laufrades (234) vorgesehene sekundäre Beschaufelung (261, 260) bewirkt bei Drehbewegungen des Laufrades (234) im Betrieb der Blutpumpe, dass die Spülströmung (252) infolge der einwirkenden Zentrifugaleffekte über die Spülkanäle (260) radial nach außen befördert wird und von dort wieder in die Hauptströmung (250) mündet.

Die axiale Lagerung des Pumpenlaufrades (234) im Pumpengehäuse (230) erfolgt nun derart, dass die Spülströmung (252) gleichzeitig als Lagerströmung für ein hydrodynamisches Axiallager (262, 240) auf der Laufradrückseite (259) verwendet wird. Die Funktionsweise dieses hydrodynamischen Axiallagers beruht in einer bevorzugten Ausführung der Blutpumpe auf einer Keilflächenlagerung (262, wie dies in Fig. 12 verdeutlicht ist. Es sind darüber hinaus auch alternative hydrodynamische Axiallager denkbar, die z.B. auf einem Spiralrillenlager beruhen.

Im Falle eines Mehrflächen-Keillagers, wie dies in Fig. 12 wiedergegeben ist, entsteht bei Rotation des Laufrades (234) eine Quetschströmung in den keilförmigen Zwischenräumen (268), welche gemäß dem "Reynolds-Effekt" eine hydrodynamische Tragkraft in axialer Richtung aufbaut, die der belastenden Kraft aus der Magnetkupplung (237, 238) entgegengerichtet ist und ein axiales Abheben des Laufrades (234) vom stationären Pumpenelement (240) hervorruft. Auf diese Weise wird eine berührungslose, hydrodynamische Axiallagerung des Laufrades (234) im Pumpengehäuse (230) gewährleistet.

Für die radiale Stabilisierung des Laufrades (234) im Pumpengehäuse (230) wird schließlich die hydrostatische Rückstellkraft gemäß dem "Lomakin-Effekt" genutzt, welche bei radialen Auslenkungen des Laufrades (234) im Pumpengehäuse (230) in der Leckageströmung (253) durch den Spaltraum (258) zwischen der Deckscheibe (245) und dem gegenüberliegenden Pumpengehäuse (230) wirksam wird, wie dies bereits bei der Ausführung der Blutpumpe gemäß Fig. 1 und Fig. 2 ausführlich beschrieben wurde.

Weiterhin wird aufgrund der radialen Rückstellkräfte, die in der axialen Magnetkupplung (237, 238) bei radialen Auslenkungen des Laufrades (234) wirksam werden, die Radialstabilität der Laufrades (234) im Pumpengehäuse (234) zusätzlich erhöht. Diese permanentmagnetische radiale Rückstellkraft ist bei der vorliegenden Erfindung ein effektives Mittel zur Zentrierung des Laufrades (234) im Pumpengehäuse und unterstützt das hydrostatische Radiallager (253, 258) in seiner Lagerfunktion.

Die wesentlichen Vorteile einer derartigen Rotorlagerung im Vergleich zu konventionellen hydrodynamischen Rotorlagern in Blutpumpen liegen einerseits in der Vereinfachung des Lagermechanismus bei Reduzierung der für die Rotorlagerung erforderlichen Bauteile, sodass die Rotorlagerung gemäß der Ausführung aus Fig. 9 bis Fig. 12 wesentlich einfacher realisiert werden kann. Andererseits ist auch der Vorteil gegeben, dass außer der hydrodynamischen Axiallagerung keine zusätzlichen Designvorkehrungen für die Rotorlagerung des Pumpenlaufrades (234) erforderlich sind. Die Radiallagerung beruht vielmehr auf Komponenten - wie Deckscheibe (245) und magnetische Axialkupplung (237, 238) - und Strömungsführungen -d.h. Leckageströmung (253) - die ohnehin als Komponenten für eine hämatologisch konforme Blutpumpe erforderlich wären. Im Vergleich zu mechanischen Rotorlagern mit Festkörperreibung und Verschleiß in den Lagerflächen liegt der Vorteil einer solchen Rotorlagerung in ihrer berührungslosen Laufradstabilisierung ohne Reibung und Verschleiß, sodass die Lebensdauer der Blutpumpe entsprechend erhöht werden kann. Durch die Verwendung eines hydrostatischen Radiallagers, welches im Gegensatz zu hydrodynamischen Lagern mit einer wesentlich größeren Lagerspaltbreite betrieben werden kann, resultiert schließlich auch der Vorteil einer solchen Rotorlagerung, dass diese nämlich im Vergleich zu einer beiderseits (radial und axial) hydrodynamisch gelagerten Blutpumpe wesentlich blutschonender betrieben werden kann, womit schließlich auch die Belastung des Blutes und damit des Patienten weitgehend minimiert werden kann.

Figur 13 zeigt eine Blutpumpe, bei der der gleiche Aufbau und die gleiche Funktionsweise der Blutpumpe vorliegt wie in Fig. 9. Bei Fig. 13 liegt dennoch eine Ausführung der Blutpumpe vor, bei der im Gegensatz zu der Ausführung aus Fig. 9 ein elektromagnetsicher Antrieb (285, 286, 287) verwendet wird, welcher außerhalb des Pumpengehäuses (280) positioniert und an der Rückseite des Pumpengehäuses (295) befestigt ist. Die Vorteile einer derartigen Ausführung wurden bereits bei der Ausführung der Blutpumpe gemäß Fig. 4 ausführlich beschrieben und können bei der Ausführung der Blutpumpe gemäß Fig. 13 unmittelbar übernommen werden.

Die Wirkungsweise der elektromagnetischen Axialkupplung (286, 287, 293) sowie die damit verbundenen Vorteile entsprechen ebenfalls denen der Ausführung gemäß Fig. 4 und haben demnach auch für die Ausführung der Blutpumpe gemäß Fig. 13 Gültigkeit.

Die Wirkungsweise der hydrostatisch-hydrodynamischen Rotorlagerung (292, 296) sowie die damit verbundenen Vorteile können entsprechend aus der Ausführung gemäß Fig. 9 übernommen werden.

Die weitere Funktionsweise der Blutpumpe insbesondere die magnetische Kupplungsvorrichtung (287, 293) sowie die sekundären Strömungsformen in der Blutpumpe können weitgehend von der Ausführung gemäß Fig. 9 übernommen werden.

Figur 14 zeigt eine Blutpumpe, bei der der Antrieb des Pumpenlaufrades (328) über eine diagonal wirkende elektromagnetische Kupplung (325, 327, 335, 333) gewährleistet wird. Die hydrodynamische Lagerung des Pumpenlaufrades (328) im Pumpengehäuse (320) erfolgt hierbei ebenfalls in diagonaler Richtung und kombiniert im Gegensatz zu der Ausführung der Blutpumpe gemäß Fig. 9 und Fig. 13 die Radiallagerung und Axiallagerung des Laufrades (328) in einer Einheit, wobei die hydrostatische Radiallagerung (332) sowie die radialen Rückstellkräfte in der Magnetkupplung (333, 335) eine zusätzliche Stabilisierung des Laufrades (328) in radialer Richtung gewährleisten, welche beispielsweise bei ungenauer Fertigung der hydrodynamischen Lagerungskomponenten eine zusätzliche Sicherheit für Rotorlagerung des Pumpenlaufrades (328) im Pumpengehäuse (320) gewährleisten.

Bezüglich der Strömungsführung einer derartigen Blutpumpe mit diagonaler Magnetkupplung gelten die gleichen Vorteile wie die der Ausführung der Blutpumpe gemäß Fig. 7.

Bezüglich der Wirkungsweise des hydrodynamischen Lagers (336) gelten weitgehend die gleichen Aspekte wie die der Ausführung gemäß Fig. 9 und Fig. 13

Figur 15 und Figur 16 zeigen eine Blutpumpe, bei der die Rotorlagerung des Pumpenlaufrades (411) im Pumpengehäuse (400) auf einer Kombination aus mechanischer und permanentmagnetischer Lagerung beruht . Der Antrieb des Laufrades (12) erfolgt über einen Elektromotor (406) und eine permanentmagnetische Axialkupplung (407, 408, 409, 410), welche in ihrem Aufbau und ihrer Funktionsweise der Ausführung der Blutpumpe aus Fig. 1 und Fig. 2 entspricht. Die zuvor erwähnten Vorteile eines derartigen Antriebskonzeptes gelten daher ebenfalls für die Ausführung der Blutpumpe gemäß Fig. 15 und Fig. 16.

Bezüglich der Strömungsführung innerhalb der Blutpumpe gelten die im Abschnitt "Aufbau und Funktionsweise der Blutpumpe" angeführten Beschreibungen und Erläuterungen. Darüber hinaus kommt bei der Blutpumpe gemäß Fig. 15 und Fig. 16 keine Deckscheibe zum Einsatz, sodass die Leckageströmung zwischen den Laufradschaufeln (412) und dem gegenüberliegenden Pumpengehäuse zumindest für die Ausführung der Blutpumpe gemäß Fig. 15 und Fig. 16 unbedeutend bleibt.

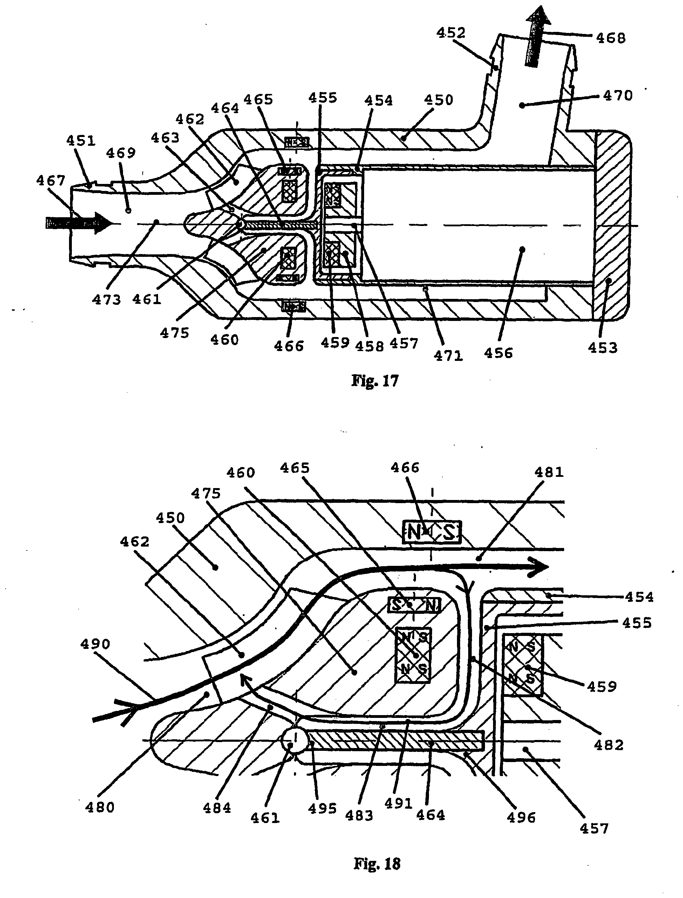

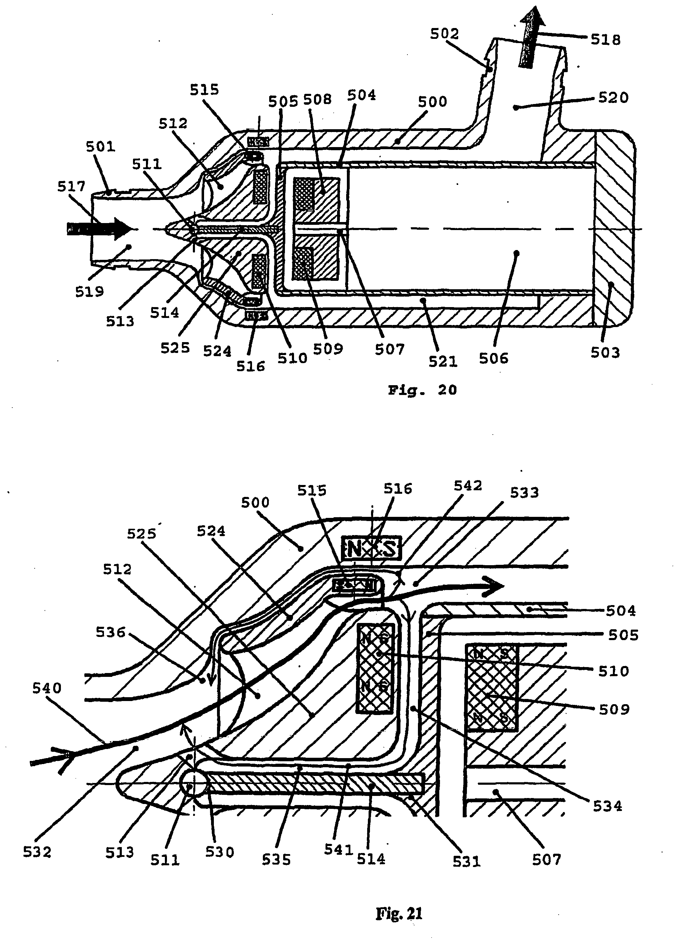

Die Ausführung der Blutpumpe gemäß Fig. 15 und Fig. 16 sowie die im Folgenden zu beschreibende Blutpumpe gemäß Fig. 17 und Fig. 18 ist dennoch auch bei Verwendung einer Deckscheibe funktionstüchtig. Die Deckscheibe hat lediglich bei der Funktionalität der Blutpumpe und insbesondere ihrer Rotorlagerung nur eine untergeordnete Bedeutung und wird von daher an dieser Stelle vernachlässigt. Bei der Blutpumpe gemäß Fig. 20 wird weiterhin auch eine Ausführung der Blutpumpe mit Deckscheibe beschrieben, die in den folgenden Abschnitten noch näher betrachtet wird.

Die mechanische Rotorlagerung umfasst bei der Blutpumpe gemäß Fig. 15 und Fig. 16 eine Kugelspurlagerung bzw. ein Spitzenlager (engl. pivot bearing) (405, 414), welche aus einer Kugel (bzw. einem Rotationskörper mit verrundeter Spitze) (414) und einer Kalotte (405) als Lagerelemente besteht. Eines der Lagerelemente stellt hierbei die rotierende Lagerkomponente dar, welche mit dem Laufradkörper (411) fest verbunden ist, und das andere Element, die stationäre Lagerkomponente, welche mit dem stationären Pumpenelement (405) fest verbunden ist, bzw. ein einheitliches Bauteil darstellt.

In einer bevorzugten Ausführung der Blutpumpe stellt die Kugel (414) die rotierende und die Kalotte (405) die stationäre Lagerkomponente dar. Als Werkstoffe der einzelnen Lagerkomponenten kommen jeweils Biokeramiken wie Aluminiumoxyd (Al203) oder Zirkoniumoxyd (ZrO2) sowie biokompatible Kunststoffe wie Ultra-Hoch-Molekulares-Polyethylen (UHMW-PE), Polyetheretherketon (PEEK), Polyoxymethylen (POM) oder Polyimid (PI) sowie biokompatible Metalle zum Einsatz. In einer bevorzugten Ausführung der Blutpumpe kommen für die rotierende Lagerkomponente Biokeramiken oder biokompatible Metalle und für die stationäre Lagerkomponente Biokeramiken oder biokompatible Kunststoffe zum Einsatz.

Die axialen Anziehungskräfte in der Magnetkupplung (409, 410) werden bei der Blutpumpe gemäß Fig. 15 und Fig. 16 durch das Pivot-Lager aufgenommen. Die Pivot-Lagerung als Kugelspurlagerung stellt hierbei gleichzeitig ein zentrierendes Radiallager dar. Aufgrund der punkthaften Wirkung des Pivot-Lagers (405, 414) ist das Laufrad (411) dennoch Kippbewegungen um das Pivotlager ausgesetzt, welche insbesondere aus den instabilen Anziehungskräften der axialen Magnetkupplung (409, 410) resultieren. Zur Stabilisierung des Laufrades (411) gegen Kippauslenkungen bedarf es daher eines Stützlagers, welches die Kippbewegungen des Laufrades (411) im Pumpengehäuse (400) eingrenzt.

Bei der Ausführung der Blutpumpe gemäß Fig. 15 und Fig. 16 wird dieses Stützlager durch ein permanentmagnetisches Lager (415, 416) hervorgebracht, bei dem ein permanentmagnetisches Element als Statormagnet (416) im Pumpengehäuse (400) und ein weiteres permanentmagnetisches Element als Rotormagnet (415) im Laufradkörper integriert ist. In einer bevorzugten Ausführung der Blutpumpe bestehen die permanentmagnetischen Elemente aus konzentrisch angeordneten dauermagnetischen Ringen, die jeweils entgegengerichtet in axialer Richtung magnetisiert sind. Als Werkstoffe kommen für diese Ringmagnete (415, 416) bevorzugt Seltenerdmagnete wie NdFeB oder SmCo zum Einsatz.

In einer bevorzugten Ausführungsform der Blutpumpe sind die einzelnen Ringmagnete (415, 416) konzentrisch zueinander angeordnet. Bei seitlichen Kippauslenkungen des Rotormagneten (415) im Statormagneten (416) wird infolge der zwischen den Ringmagneten (415, 416) wirksamen axialen Rückstellkräfte ein stabiles Rückstellmoment hervorgerufen, welches den Rotormagneten (415) wieder in die unausgelenkte Lage versetzt. Auf diese Weise ist für die Blutpumpe gemäß Fig. 15 und Fig. 16 ein stabiles Stützlager gegen seitliche Kippbewegungen des Pumpenlaufrades (411) im Pumpengehäuse (400) definiert. Die Rotorlagerung des Pumpenlaufrades (411) beruht somit auf einer Mischform-Lagerung ("Hybridlager"), basierend auf der Kombination einer mechanischen Pivot-Lagerung (405, 414) mit einer permanentmagnetischen Stützlagerung (415, 416).

Die axiale Position der einzelnen konzentrischen Ringmagnete (415, 416) zueinander sowie ihre axiale Position relativ zur Pivot-Lagerung (405, 414) sind bei der Blutpumpe gemäß Fig. 15 und Fig. 16 sowie den weiteren Lagerungsvarianten, die im Folgenden noch beschrieben werden, von zentraler Bedeutung für die Stabilität der Rotorlagerung.

Bei der Ausführung der Blutpumpe gemäß Fig. 15 und Fig. 16 befindet sich das Pivot-Lager (405, 414) (Kugelmittelpunkt) auf gleicher axialer Höhe wie der Rotormagnet (415). Der Statormagnet (416) befindet sich hierbei ebenfalls auf gleicher axialer Höhe wie der Rotormagnet (415). Diese Konstellation reicht bereits aus, um eine weitgehend stabile Lagerung des Pumpenlaufrades (411) im Pumpengehäuse (400) zu gewährleisten.

Es ist hierbei zu berücksichtigen, dass die Pivot-Lagerung (405, 414) nur ein einseitig wirksames axiales Drucklager darstellt und die Lagerkugel (414) daher stets in der Kalotte (405) verweilen muss, da bei Ausgleiten der Lagerkugel (414) aus der Kalotte (414) infolge der instabilen radialen Anziehungskräfte zwischen dem Rotormagneten (415) und dem Statormagneten (416) keine radiale Zentrierung mehr für das Laufrad (411) im Pumpengehäuse (400) gegeben ist. Eine axial versetzte Platzierung des Statormagneten (416) in Richtung des Pumpeneinlasses bringt beispielsweise aus diesen Gründen keine sinnvolle Rotorlagerung unter den vorliegenden Gegebenheiten hervor.

Ein Ausgleiten der Pivot-Lagerung (405, 414) wird zudem durch die im Betrieb der Pumpe auf das Laufrad (411) einwirkenden hydraulischen Strömungskräfte (Axialschub) begünstigt, welche ein Abheben des Laufrades (411) von der Pivot-Lagerung (405, 414) hervorrufen. Diese Gefahr des Ausgleitens und Abhebens stellt im gegenwärtigen Stand der Technik zu Blutpumpen mit Pivot-Lagerung als einziges Axiallager ein erhöhtes Sicherheitsrisiko für den Betrieb der Pumpe dar, da bei Ausgleiten der Pivot-Lagerung nicht nur eine unerwünschte Lärmemission infolge der unkontrollierten Rotorbewegungen im Pumpengehäuse einhergeht, sondern auch unter Umständen der Fluss- und Druckaufbau als Hauptfunktion der Blutpumpe wesentlich beeinträchtigt wird.

Der Gefahr des Ausgleitens wird im gegenwärtigen Stand der Technik durch die axialen Anziehungskräfte der Magnetkupplung entgegengewirkt. Dennoch kann dies bei extremen Betriebspunkten mit hohem Druckaufbau in der Blutpumpe dazu führen, dass die hydraulischen Strömungskräfte betragsmäßig die Anziehungskräfte der Magnetkupplung übersteigen und somit keine stabile Lagerung des Laufrades im Pumpengehäuse mehr gegeben ist.

Gemäß Fig. 15 und Fig. 16 hingegen wird infolge der axialen Rückstellkräfte in der Magnetlagerung (415, 416) neben einem Stützlager gegen Kippauslenkungen zusätzlich auch ein wechselseitig wirkendes Axiallager zur Verfügung gestellt, sodass hiermit die Gefahr des Ausgleitens der Pivot-Lagerung effektiv unterbunden wird.

Eine weitere vorteilhafte Besonderheit der Blutpumpe gemäß Fig. 15 und Fig. 16 liegt darin, dass bei Beibehaltung der konzentrischen Anordnung der Magnetlagerung (415, 416) und der Pivot-Lagerung eine höhere Rotorstabilität des Laufrades (411) erreicht werden kann, wenn der Statormagnet (416) axial um einen bestimmten Abstand in Richtung der Magnetkupplung (409, 410) versetzt positioniert wird, wie dies aus Fig. 16 zu erkennen ist. Der axiale Abstand der Versetzung des Statormagneten (416) gegenüber dem Rotormagneten (415) beträgt hierbei zwischen 1% und 50%, in einer bevorzugten Ausführung zwischen 5 % und 25%, der axialen Abmessung des Statormagneten (416).

Die in der Magnetlagerung (415, 416) wirksamen Axialkräfte werden hierbei als Vorspannung in der Pivot-Lagerung (405, 414) aufgefangen. Durch eine derartige Positionierung des Statormagneten (416) wird einerseits infolge der vorgespannten Axialkräfte zwischen dem Rotormagnet (415) und dem Statormagnet (416) die Kippsteifigkeit des Magnetlagers (415, 416) weiter erhöht, wodurch die seitlichen Kippbewegungen weiter eingegrenzt und die Rotorstabilität damit erhöht werden können. Andererseits bringt eine derartige Konstellation auch gleichermaßen ein permanentmagnetisches Axiallager mit erhöhter Lagersteifigkeit für das Pumpenlaufrad (411) hervor, welches das Risiko des Ausgleitens des Pumpenlaufrades (411) aus der Pivot-Lagerung (405, 406) weiter eingrenzt und somit die Rotorstabilität auch bei jenen extremen Applikationen aufrechterhält, die durch einen hohen Druckaufbau in der Blutpumpe gekennzeichnet sind. Bei extrakorporalen Anwendungen der Blutpumpe im Rahmen einer Herz-Lungen-Maschine (HLM) oder im Rahmen eines ECMO-Systems mit erforderlichen Druckaufbauten von bis zu 800 mmHg kann ein derartiges Lagerungskonzept effektiv und aufgrund der einfachen Bauweise der Blutpumpe mit geringem Herstellungsaufwand eingesetzt werden.

Die Umspülung der Laufradrückseite (427) sowie der Pivot-Lagerung (405, 414) wird hierbei mit Hilfe der bereits in Fig. 1 beschriebenen Spülströmung (429) und den dafür im Laufradkörper (411) vorgesehenen Spülkanälen (413) gewährleistet, sodass einerseits Strömungsstagnationen auf der Laufradrückseite (427) vermieden werden und andererseits die Reibungswärme der Pivot-Lagerung (405, 414) effektiv abgeführt wird und die Blutpumpe schließlich in ihrer Gesamtheit blutschonend betrieben werden kann.

Figur 17, Figur 18 und Figur 19 zeigen eine Blutpumpe, bei der die Rotorlagerung des Pumpenlaufrades (475) im Pumpengehäuse (450) auf einer Kombination aus mechanischer und permanentmagnetischer Lagerung beruht, wobei im Gegensatz zur Ausführung der Blutpumpe gemäß Fig. 15 und Fig. 16 die mechanische Lagerung (461, 495) auf ungleicher axialer Höhe zum permanentmagnetischen Lager (465, 466) positioniert ist.

Der Aufbau und die Funktionsweise dieser Ausführung entsprechen weitgehend der Blutpumpe aus Fig. 15 und Fig. 16. Dies betrifft insbesondere die Antriebseinheit (456, 457, 458, 459), die Kupplungsvorrichtung (457, 458, 459, 460), die Strömungsführung innerhalb der Pumpe sowie den Aufbau und die Funktionsweise der Magnetlagerung (465, 466).

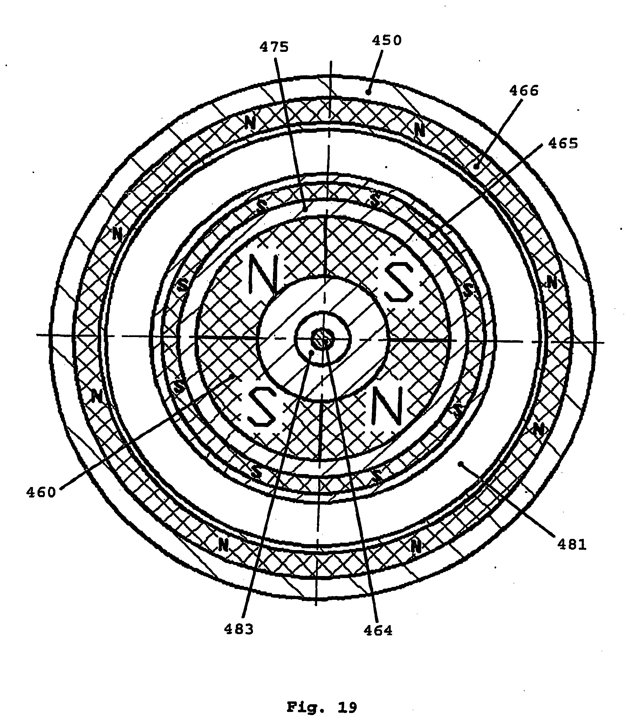

Die Abbildung Fig. 19 zeigt hierzu als Schnittbild durch die Blutpumpe die Magnetisierung der einzelnen Kupplungs- und Lagermagnete.

Die zuvor genannten Vorteile in Bezug auf die o.g. Pumpenkomponenten sind daher auch für die Ausführung der Blutpumpe gemäß Fig. 17 gültig.

Bei der Ausführung der Blutpumpe gemäß Fig. 17, Fig. 18, und Fig. 19 ist dennoch eine veränderte Ausführung der Rotorlagerung gezeigt, bei der die axiale Position des mechanischen Pivot-Lagers (461, 495) gegenüber der permanentmagnetischen Stützlagerung (465, 466) axial in Richtung des Pumpeneinlasses (451) versetzt ist. Die Pivot-Lagerung (461, 495) basiert auch hier auf einer mit dem Laufradkörper (475) verbundenen Lagerkugel (461) und einer Kalotte (495), die über eine verlängerte Achse (464) mit dem stationären Pumpenelement (455) verbunden ist.

Die Funktionsweise der Pivot-Lagerung (461, 495) entspricht im Wesentlichen der Funktionsweise der Pivot-Lagerung aus Fig. 15 und Fig. 16. Gleiches gilt auch für die Wahl der Werkstoffe zu den einzelnen Lagerkomponenten.

Bei der Blutpumpe gemäß Fig. 17, Fig. 18 und Fig. 19 erfolgt die Stabilisierung des Laufrades (475) gegen seitliche Kippbewegungen um das Pivot-Lager (461, 495) ebenfalls mit Hilfe einer permanentmagnetischen Stützlagerung (465, 466), bei der der Rotormagnet (465) im Laufradkörper (475) integriert und der Statormagnet (466) im Pumpengehäuse (450) eingebettet ist. Bezüglich der Wahl der Magnetwerkstoffe gelten die gleichen Angaben wie bei der Blutpumpe aus Fig. 15 und Fig. 16.

Der wesentliche Unterschied der Blutpumpe aus Fig. 17 im Vergleich zur Blutpumpe aus Fig. 15 liegt darin, dass infolge der unterschiedlichen axialen Position des Magnetlagers (465, 466) und des Pivot-Lagers (461, 495) die Magnetlagerung (465, 466) bei der Stabilisierung des Laufrades (475) gegen Kippbewegungen mit einem wesentlich größeren Hebelarm (entspricht dem axialen Abstand zwischen Magnetlager und Pivot-Lager) arbeitet und somit die für die Stabilisierung des Laufrades (475) erforderlichen magnetischen Rückstellmomente bei gleichen magnetischen Kraftverhältnissen zwischen dem Rotormagneten (465) und dem Statormagneten (466) wesentlich erhöht werden können und die Stabilität der Rotorlagerung entsprechend zunimmt.

Die blutschonende Förderung wird auch bei der Blutpumpe gemäß Fig. 17 weiterhin durch die Umspülung der Laufradrückseite (482) sowie insbesondere die Umspülung der Pivot-Lagerung (461, 495) mit Hilfe der Spülströmung (491) aufrechterhalten.

Zur weiteren Erhöhung der Sicherheit gegen Ausgleiten des Pumpenlaufrades (475) aus der Pivot-Lagerung (461, 495) besteht weiterhin bei der Blutpumpe gemäß Fig. 17 die Möglichkeit, eine axial versetzte Positionierung des Statormagneten (466) gegenüber dem Rotormagneten (465) zu realisieren, sodass die Stabilisierung des Laufrades (475) bei gleichzeitiger Minimierung der Lagerbelastung in der Pivot-Lagerung (461, 495) durch Anpassen der einzelnen axialen Abstände den jeweiligen klinischen Anforderungen angepasst werden kann.

Figur 20 und Figur 21 zeigen eine Blutpumpe, die in ihrem Aufbau und ihrer Funktionsweise weitgehend der Blutpumpe aus Fig. 17 entspricht. Die Positionierung der Magnetlägerung (515, 516) erfolgt jedoch bei der Blutpumpe gemäß Fig. 20 nicht im Laufradkörper (525) selbst, sondern in der Deckscheibe (524). Hierdurch bietet sich eine weitere Möglichkeit an, die Tragkraft des magnetischen Stützlagers (515, 516) und damit auch die Stabilität des Laufrades (525) weiter zu erhöhen, da der Luftspalt zwischen Rotormagnet (515) und Statormagnet (516) im Vergleich zur Blutpumpe aus Fig. 17 verkleinert ist und die in der Magnetlagerung (515, 516) wirksamen Lagerkräfte somit entsprechend erhöht werden.

Der weitere Aufbau und die Funktionsweise entsprechen weitgehend denen der Blutpumpe aus Fig. 17, sodass die jeweiligen Vorteile an dieser Stelle übernommen werden können.

Figur 22 bis Figur 31 zeigen eine Blutpumpe, die in ihrem Aufbau der Blutpumpe aus Fig. 20 sehr ähnlich ist. Dennoch liegt bei der Blutpumpe gemäß Fig. 20 der wesentliche Unterschied darin, dass zur Stabilisierung des Laufrades (625) gegen Kippbewegungen um die Pivot-Lagerung ein magnetisch wirkendes Lager zum Einsatz kommt, welches bereits in der verwendeten Diagonalkupplung implementiert ist. Demnach liegt hierbei die Ausnutzung eines Synergieeffektes zwischen der Kupplungsvorrichtung und der Lagerungsvorrichtung vor, die im Wesentlichen dazu führt, dass der Bedarf nach einer separaten Magnetlagerung für die Kippstabilisierung des Laufrades entfällt und die magnetische Diagonalkupplung sowohl zu Antriebszwecken als auch zu Lagerungszwecken verwendet wird.

Durch eine derartige Ausführung wird die Blutpumpe einerseits einfacher in ihrem Aufbau und in ihrer Funktionsweise, wodurch die Betriebssicherheit der Blutpumpe entsprechend erhöht wird. Andererseits wird die Blutpumpe in ihrer Gesamtheit wesentlich kompakter, sodass auch Vorteile hinsichtlich der Implantierbarkeit einer solchen Blutpumpe hervorgehen.

Die vorteilhaften Eigenschaften einer Diagonalkupplung hinsichtlich der Strömungsführung sowie weiterer Aspekte können aus der Blutpumpe gemäß Fig. 7 und Fig. 14 übernommen werden. Die Abbildung Fig.23 zeigt hierbei eine Detailansicht zur Strömungsführung in der Blutpumpe gemäß Fig. 22

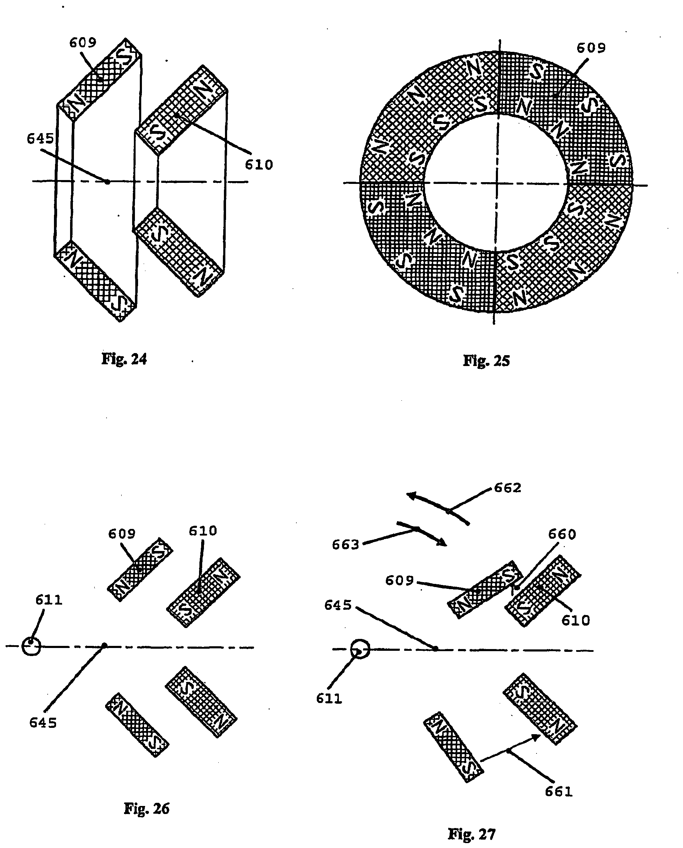

Die Abbildungen Fig. 24 bis Fig. 27 zeigen den Aufbau und die Funktionsweise der in der Diagonalkupplung integrierten Rotorlagerung bei Kippbewegungen des Laufrades um die Pivot-Lagerung. Hierbei sind die einzelnen Magnetsegmente in radialer Richtung einfach ausgeführt.

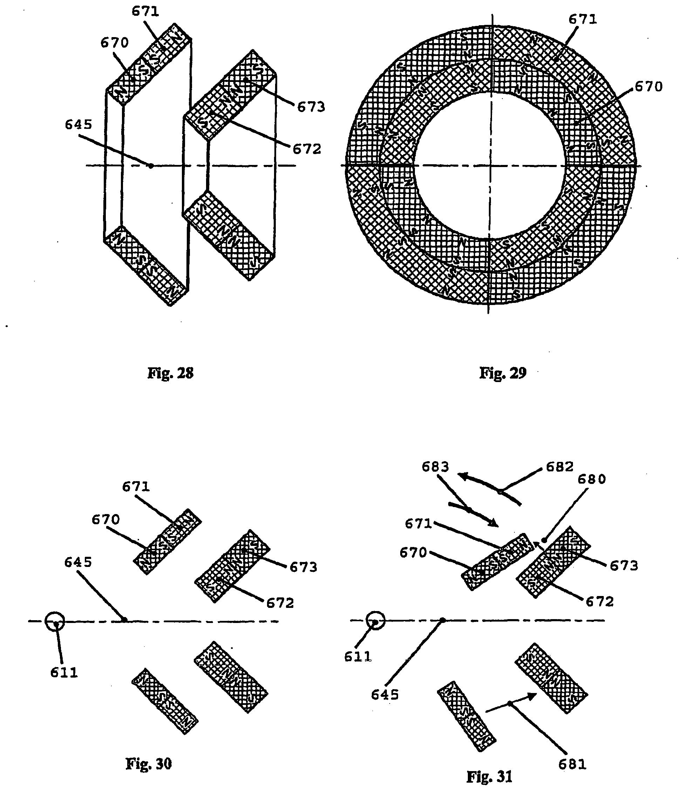

Eine Teilung der Magnetsegmente in radialer Richtung sowie die daraus resultierende Funktionsweise ist in den Abbildungen Fig. 28 bis Fig. 31 verdeutlicht. Infolge der Teilung der Kupplungsmagnete in radialer Richtung sowie das Zusammenfügen von zwei einzelnen Magnetsegmenten an ihren radialen Enden in gegensinniger Polarisierungsrichtung hat zur Folge, dass die magnetischen Feldlinien entlang der Teilungslinie verdichtet werden, wodurch sowohl höhere Drehmomente übertragen werden können als auch der Stabilisierungseffekt der Diagonalkupplung verstärkt wird, wie dies insbesondere in Fig. 31 zu ersehen ist.

Durch eine derartige Teilung der Kupplungsmagnete lässt sich somit die Betriebssicherheit der Blutpumpe effektiv erhöhen, wobei der Produktionsaufwand eines derartigen Systems aufgrund der einfachen und unkomplexen Bauteile entsprechend gesenkt werden kann.

Diese Vorteile stehen insbesondere in Konformität mit den Anforderungen der medizinischen Technologie hinsichtlich höchster Zuverlässigkeit und maximaler Aufwandsreduzierung der zu verwendenden medizintechnischen Produkte.

Figur 32 bis Figur 35 zeigen eine Blutpumpe, die unabhängig vom gewählten Rotorlagerungs-konzept eine äußere Bauform aufweist, die einer "harmonischen Flachbauweise" der Blutpumpe gleichkommt. Unter einer "harmonischen Flachbauweise" ist eine Pumpengeometrie zu verstehen, deren Querschnittsabmessung senkrecht zur Drehachse (906) ein Höhen-Breiten-Verhältnis von H/B < 1 aufweist. Der wesentliche Vorteil einer derartigen Ausführung liegt in der einfacheren Implantierbarkeit der Blutpumpe in entsprechenden Bereichen der menschlichen Anatomie. Insbesondere bei subkutaner Platzierung der Blutpumpe, welche in der Medizintechnik im Hinblick auf "Minmal Invasive Systeme" bevorzugt zur Anwendung kommt, können enorme klinische Vorteile erzielt werden, die sich im Wesentlichen zusammenfassen lassen als:
- patientenfreundlich, da minimaler chirurgischer Eingriff
- leichter explantierbar bei temporärer Anwendung über mittelfristige Einsatzdauer wie z.B. dem Bridge-to-Recovery-Concept
- leichter einer Diagnoseprüfung der Pumpe zugänglich, insbesondere bei kritischem Pumpenbetrieb und leichterer Austausch der Pumpe durch neue Blutpumpe möglich

In den Abbildungen Fig. 32 bis Fig. 35 ist beispielhaft zu einer harmonischen Flachbauweise des Pumpengehäuses (900) eine Blutpumpe mit elliptischem Querschnitt wiedergegeben, wobei der dem Laufrad (905) zugeordnete Bereich des Pumpengehäuses (901) - zumindest im jeweiligen Innenraum des Pumpengehäuses - kreiszylindrisch, der dem Antrieb zugeordnete Bereich (910) jedoch elliptisch ausgeführt werden kann. Im Falle der Verwendung einer permanentmagnetischen Kupplungsvorrichtung kann weiterhin der für die Kupplungsvorrichtung vorgesehene Bauraum (911) - zumindest im jeweiligen Innenraum des Pumpengehäuses - kreiszylindrisch ausgeführt werden.

Insbesondere die Ausführung des dem Antrieb zugeordneten Gehäusebereichs (910) als elliptischer Querschnitt bringt bei Beibehaltung der o.g. Vorteile einer subkutan implantierbaren Blutpumpe, vorteilhaft bezüglich des Antriebs, insbesondere auf Basis eines Elektromotors, die auf einer höheren Leistungsfähigkeit der elliptischen Pumpe im Vergleich zu einer kreiszylindrischen Pumpe mit vergleichbarem Durchmesser beruhen.

Der wesentliche Vorteil einer Antriebseinheit in harmonischer Flachbauweise liegt darin, dass bei einem Elektromotor mit beispielsweise elliptischem Querschnitt, wie dies in den Abbildungen Fig. 34 und Fig. 35 wiedergegeben ist, die Leistungsfähigkeit der Pumpe im Vergleich zu anderen Pumpen mit vergleichbarem Durchmesser relativ größer ist. Geht man beispielsweise von einem elliptischen Pumpen- und Motordesign aus, wobei die Ellipse die Hauptabmessungen Breite B und Höhe H aufweist, mit H<B und setzt demgegenüber eine konventionelle kreiszylindrische Pumpenausführung mit Durchmesser D zum Vergleich, d.h. H = D (kleinste Abmessungen als relevant für Zugänglichkeit, Tunnelbarkeit bei der Implantationsprozedur, besser geeignet für subkutane Anwendungen) so wird die elliptische Ausführung des Motors im Vergleich zur kreiszylindrischen Ausführung aufgrund der effektiveren Nutzung der elliptischen Bauräume für Motorwicklungen (932, 942) und Statoreinheiten (931, 941) folgende Vorteile aufweisen:
- Erzeugen eines höheren Maximal-Drehmoments und damit auch Erweitern des realisierbaren Betriebsbereiches der Pumpe.
- Benötigen eines niedrigeren Stromverbrauchs und damit eines geringeren Leistungsbedarfes der Blutpumpe (Batteriebetrieb insbesondere von Relevanz hierzu)
- Höherer Gesamtwirkungsgrad und damit effizienteres Arbeiten der Blutpumpe.

Diese Vorteile lassen sich anders auch formulieren als
- Eine Blutpumpe mit elliptisch kleinerem Durchmesser (H<D) bringt die gleiche hämodynamische Leistungsfähigkeit hervor wie eine kreiszylindrische Pumpe mit großem Durchmesser.

Insbesondere bei Axialblutpumpen und Diagonalblutpumpen, die vorwiegend eine axial längliche Bauform aufweisen, entstehen mit einer derartigen Ausführung der Blutpumpe sowohl Vorteile hinsichtlich der Leistungsfähigkeit der Blutpumpe als auch Vorteile, die von klinischer Relevanz für einen patientenschonenden Betrieb der Blutpumpe sind.

Figur 36 zeigt eine Ausführung, bei der die Blutpumpe als separater Pumpenkopf (1000) von der Antriebseinheit (1050, 1070, 1071, 1072) getrennt werden kann, sodass die Antriebseinheit (1050, 1070, 1071, 1072) als verhältnismäßig herstellungsaufwendige Komponente des Blutpumpensystem wieder verwendbar ist, während der Pumpenkopf (1000) als leicht herstellbares Einwegteil (engl. Disposable) nach jedem klinischen Einsatz ausgetauscht werden kann. Diese Ausführung bietet insbesondere bei extrakorporalen Anwendungen (HLM, ECMO, etc.) über eine kurz- bis mittelfristige Einsatzdauer entsprechende Vorteile.

Als Pumpenkopf (1000) eignen sich hierbei jeweils alle jenen Ausführungen der Blutpumpe, welche insbesondere einfach produzierbar sind. Dies betrifft insbesondere die Ausführungen der Blutpumpe mit mechanischer Rotorlagerung gemäß den Abbildungen Fig. 15 bis Fig. 31. Die Möglichkeiten des erhöhten Druckaufbaus bei gleichzeitiger Gewährleistung einer ausreichenden Rotorstabilität bei der Ausführung der Blutpumpe gemäß den Abbildungen aus Fig. 15, Fig. 17 und Fig. 20 prädestinieren diese Ausführungsformen der Blutpumpen gerade für diejenigen extrakorporalen Applikationen (HLM, ECMO, etc.) bei denen ein hoher Druckaufbau in der Blutpumpe erforderlich ist.

Die Übertragung der Drehmomente von der Antriebseinheit (1050, 1070, 1071, 1072) auf das Pumpenlaufrad kann hierbei über eine permanentmagnetische oder aber auch elektromagnetische Kupplungsvorrichtung erfolgen. Die Antriebseinheit (1050, 1070, 1071, 1072) ist in ihrer Gesamtheit in einem Antriebsgehäuse (1060) untergebracht und an einem öffnungs- und schließbaren Ende zum Ein- und Ausbau der Antriebseinheit (1050, 1070, 1071, 1072) über einen Gehäusedeckel verschlossen. Diese Abdeckung der Antriebseinheit (1050, 1070, 1071, 1072) gewährleistet, dass die Antriebseinheit (1050, 1070, 1071, 1072) gegen Verunreinigungen aus der Umgebung geschützt bleibt. Zur effektiven Abfuhr der Motorwärme können an der Außenfläche des Pumpengehäuses (1060) Kühlrippen (1061) angebracht sein. In dieser Ausführungsform kann die Blutpumpe insbesondere effizient bei kurz- bis mittelfristigen Anwendungen der extrakorporalen Blutzirkulation eingesetzt werden.

Figur 37, Figur 38 und Figur 39 zeigen eine Ausführung, bei der die Blutpumpe als trennbarer Pumpenkopf (Disposable) (1100) über eine separate pneumatisch betriebene Turbine (1170) angetrieben wird.

Die Anbindung des Pumpenkopfes (1100) an die Antriebseinheit (1160) kann beispielsweise über einen Drehverschluss (1120, 1164) erfolgen, welcher ein einfaches und schnelles Trennen des Pumpenkopfes (1100) von der Antriebeinheit (1160) ermöglicht.

Die Übertragung der Drehmomente von der Turbine (1170) auf das Pumpenlaufrad (1100) erfolgt im Rahmen der Ausführung gemäß Fig. 37 bevorzugt mit Hilfe einer permanentmagnetischen Kupplungsvorrichtung (1178, 1179), welche über die Antriebswelle (1172) mit der Turbine (1170) drehstarr verbunden ist.

Die Funktionsweise der Turbine (1170) kommt im Wesentlichen einer aus dem allgemeinen Stand der Technik bekannten Gasturbine gleich, welche über Abmessungen verfügt, die dem Pumpenkopf (1100) angepasst sind.

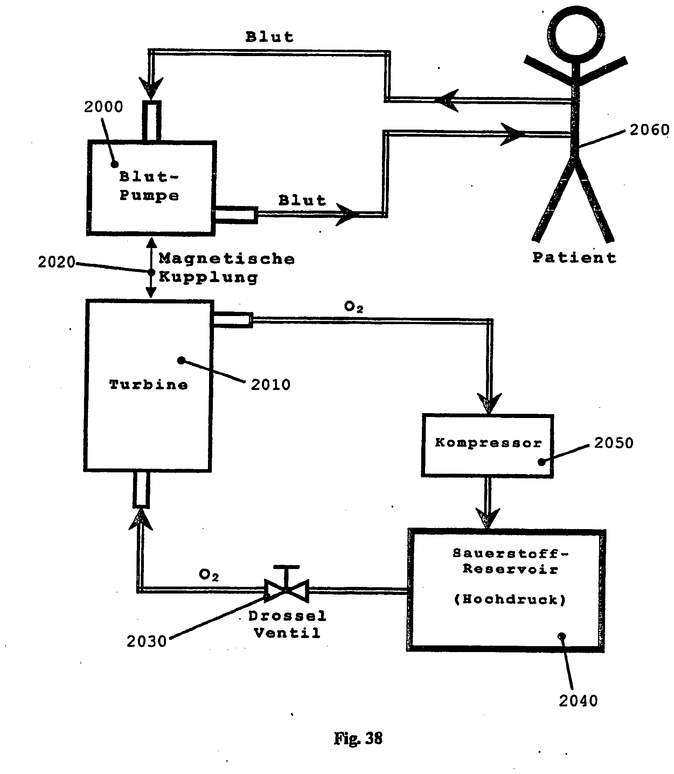

Dieses Antriebskonzept bringt insbesondere bei all jenen Anwendungen der extrakorporalen Zirkulation eine Reihe von Vorteilen hervor, bei denen die Turbine über eine Hochdruck-Sauerstoffversorgung angetrieben wird. Die Besonderheit von Sauerstoff als Antriebsenergiequelle liegt einerseits darin begründet, dass Sauerstoff in jedem OP-Raum und in nahezu jeder Klinik-Station verfügbar ist. Die Abbildung Fig. 38 illustriert hierzu schematisch den Einsatz eines derartigen Pumpen- und Antriebsystems beim Einsatz am Patienten. Die Anpassung der Laufraddrehzahl an die erforderlichen Betriebsbedingungen kann hierbei über ein manuelles oder automatisch geregeltes Ventil (2030) erfolgen. Ziel einer derartigen Anwendung wäre lediglich die extrakorporale Perfusion bestimmter Organe mit Blut, welches entweder vom Patienten selber stammt oder aber als Transfusion dem Patienten zusätzlich zugeführt wird.

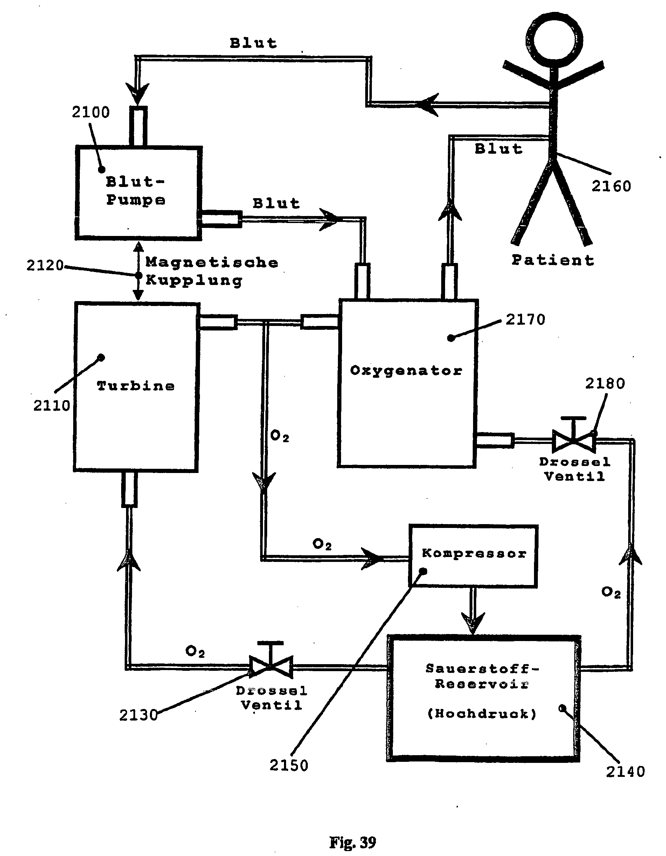

Eine weitere pragmatische Anwendung dieses Pumpen- und Antriebskonzeptes ist in Fig.39 illustriert. Hier wird durch Kombinieren der Blutpumpe mit einem Blutoxygenationssystem (Oxygenator) ein geeigneter Synergie-Effekt zunutze gemacht, bei dem Sauerstoff sowohl für die Oxygenation des Blutes als auch für den Pumpenantrieb verwendet wird. Insbesondere für kurz- bis mittelfristige Anwendungen (Herz-Lungen-Maschine, ECMO, etc.) kann ein derartiges Antriebskonzept wesentlich effizienter eingesetzt werden als konventionelle Perfusionssysteme.

Eine weitere Besonderheit bei einem derartigen Antriebskonzept liegt in der separaten Ausführung der Blutpumpe, welche ein vom Oxygenator unabhängig einsetzbares Blutfördersystem darstellt. Eine Trennung der sauerstoffangetriebenen Blutpumpe von der Oxygenatoreinheit bringt den Vorteil, dass die hier vorgestellte Blutpumpe mit jedem beliebigen Oxygenator verwendet werden kann und der Kardiotechniker somit nicht gezwungen ist, einen vom Hersteller der Blutpumpe vorgegebenen Oxygenator zu verwenden. Ein derartiges modulares Perfusionskonzept bringt insbesondere Vorteile hinsichtlich der Effizienz des Perfusionssystems.

Figur 40 zeigt den Einsatz einer Blutpumpe als implantierbares VAD-System.

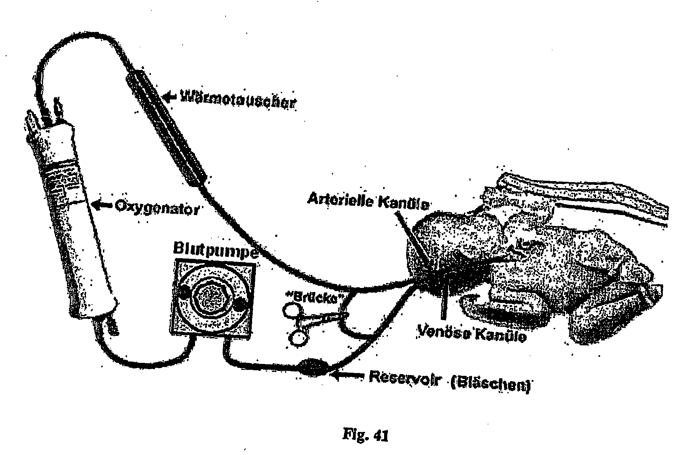

Figur 41 zeigt den Einsatz einer Blutpumpe bei der extrakorporalen Zirkulation in Kombination mit einem Oxygenator während eines ECMO-Einsatzes an einem Kleinkind.

### Bezugszeichenliste:

- 1: Pumpengehäuse
- 2: Pumpeneinlass
- 3: Pumpenauslass
- 4: Gehäusedeckel
- 5: Elektromotor
- 6: Motorwelle
- 7: Motorgehäuse
- 8: Polschuh der Magnetkupplung
- 9: Antriebsmagnete
- 10: Motordeckel, stationäres Pumpenelement
- 11: Abtriebsmagnete
- 12: Pumpenlaufrad
- 13: Laufradschaufeln
- 14: Zuströmkanal
- 15: Ringkanal zwischen Elektromotor und Pumpengehäuse
- 16: Rotormagnet der permanentmagnetischen Lagerung
- 17: Statormagnet der permanentmagnetischen Lagerung
- 18: Deckscheibe
- 19: Statoreinheit der elektromagnetischen Radiallagerung
- 20: Rotoreinheit der elektromagnetischen Radiallagerung
- 21: Blutfluss in die Pumpe hinein
- 22: Blutfluss aus der Pumpe heraus
- 23: Drehachse
- 30: Spülkanal im Laufradkörper
- 31: axiale Spaltraum auf der Rückseite des Laufrades
- 32: radiale Spaltraum der Leckageströmung
- 33: Laufradrückseite
- 34: Hauptstrom
- 35: Leckageströmung
- 36: Abzweigepunkt derSpülströmung vom Hauptstrom
- 37: Einmündung zum Leckagestromkanal
- 38: Ausmündung zum Leckagestromkanal
- 39: Spülströmung im Spülkanal
- 40: Hochdruckbereich am Laufradaustritt
- 41: Strömungskanal auf der Vorderseite des Laufrades
- 42: Spülstrom im axialen Spaltraum auf der Laufradrückseite
- 43: Laufradkörper, Laufradnabe
- 70: Pumpengehäuse
- 71: Pumpeneinlass
- 72: Pumpenauslass
- 73: Pumpenlaufrad
- 74: Abtriebsmagnete
- 75: Statormagnet des Antriebs
- 76: Wicklungen des Antriebs
- 77: Motorgehäuse
- 78: Motordeckel, stationäres Pumpenelement
- 79: Pumpendeckel
- 80: Rotormagnet der permanentmagnetischen Lagerung
- 81: Statormagnet der permanentmagnetischen Lagerung
- 82: Rotoreinheit der elektromagnetischen Radiallagerung
- 83: Statoreinheit der elektromagnetischen Radiallagerung
- 84: Ringkanal
- 100: Pumpengehäuse
- 101: Pumpeneinlass
- 102: Pumpenauslass
- 103: Pumpenlaufrad
- 104: Blutfluss in die Pumpe hinein
- 105: Blutfluss aus der Pumpe heraus
- 106: Abtriebsmagnete
- 107: Statormagnet des Antriebs
- 108: Wicklungen des Antriebs
- 109: Motorgehäuse
- 110: Statormagnet der permanentmagnetischen Lagerung
- 111: Rotormagnet der permanentmagnetischen Lagerung
- 112: Rotoreinheit der elektromagnetischen Radiallagerung
- 113: Statoreinheit der elektromagnetischen Radiallagerung
- 114: Hinterer Teil des Pumpengehäuses
- 115: Hochdruckbereich am Laufradaustritt
- 116: seitlicher Strömungsraum zwischen Laufrad und Gehäuse
- 130: Pumpengehäuse
- 131: Pumpeneinlass
- 132: Pumpenauslass
- 133: Pumpenlaufrad
- 134: Blutfluss in die Pumpe hinein
- 135: Blutfluss aus der Pumpe heraus
- 136: Abtriebsmagnete
- 137: Statormagnet des Antriebs
- 138: Wicklungen des Antriebs
- 139: Motorgehäuse
- 140: Statormagnet des Antriebs
- 141: Pumpengehäuse
- 142: Rotoreinheit der elektromagnetischen Radiallagerung
- 143: Statoreinheit der elektromagnetischen Radiallagerung
- 144: Pumpengehäuse
- 145: Hochdruckbereich am Laufradaustritt
- 146: Pumpengehäuse
- 147: seitlicher Strömungsraum zwischen Laufrad und Gehäuse
- 148: Strömungskanal im Pumpenauslass
- 149: Laufradschaufeln
- 150: Deckscheibe
- 151: Pumpenlaufrad
- 152: Spülkanal
- 153: Spülkanal
- 154: Spülkanal
- 155: Spülströmung
- 156: Spülkanal im Laufradkörper
- 157: Hauptstrom
- 158: Hauptstrom
- 159: Leckagestrom
- 160: Spülstrom
- 180: Pumpengehäuse
- 181: Pumpeneinlass
- 182: Pumpenauslass
- 183: Pumpenlaufrad
- 184: Blutfluss in die Pumpe hinein
- 185: Blutfluss aus der Pumpe heraus
- 186: Abtriebsmagnete
- 187: Statormagnet des Antriebs
- 188: Wicklungen des Antriebs
- 189: Motorgehäuse
- 190: Statormagnet des Antriebs
- 191: Pumpengehäuse
- 192: Rotoreinheit der elektromagnetischen Radiallagerung
- 193: Statoreinheit der elektromagnetischen Radiallagerung
- 194: Pumpengehäuse
- 195: Hochdruckbereich am Laufradaustritt
- 196: Pumpengehäuse
- 197: seitlicher Strömungsraum zwischen Laufrad und Gehäuse
- 198: Strömungskanal im Pumpenauslass
- 199: Laufradschaufeln
- 200: Deckscheibe
- 204: Spülkanal
- 205: Spülströmung
- 206: Spülkanal
- 207: Leckagekanal
- 208: Hauptstrom
- 209: Leckageströmung
- 210: Spülströmung
- 230: Pumpengehäuse
- 231: Pumpeneinlass
- 232: Pumpenauslass
- 233: Elektromotor
- 234: Pumpenlaufrad
- 235: Motorwelle
- 236: Polschuh
- 237: Antriebsmagnete
- 238: Abtriebsmagnete
- 239: hydrodynamisches Axiallager
- 240: Motordeckel, stationäres Pumpenelement
- 241: Motorgehäuse
- 242: Pumpendeckel
- 243: Laufradschaufeln
- 245: Deckscheibe
- 246: Strömungsraum auf der Laufradvorderseite
- 247: strömungsmechanische Radialstabilisierung
- 248: Spülkanal im Laufradkörper
- 249: Strömungskanal im Pumpeneinlass
- 250: Hauptstrom
- 251: Spülstrom
- 252: Spülstrom
- 253: Leckagestrom
- 254: Laufradvorderseite
- 255: Hochdruckbereich am Laufradaustritt Einmündung zum
- 256: Einmündung zum Leckagekanal
- 257: Ausmündung des Leckagekanals
- 258: radialer Spaltraum zwischen Laufrad und Pumpengehäuse
- 259: Strömungsraum auf der Laufradrückseite
- 260: Sekundärbeschaufelung
- 261: Strömungsraum zur Sekundärbeschaufelung
- 262: Keilflächen des hydrodynamischen Axiallagers
- 263: Drehrichtung des Laufrades
- 265: Blutfluss in die Pumpe hinein
- 266: Blutfluss aus der Pumpe heraus
- 267: Ringkanal
- 268: keilförmiger Zwischenraum der hydrodynamischen Lagerung
- 280: Pumpengehäuse
- 281: Pumpeneinlass
- 282: Pumpenauslass
- 283: Blutfluss in die Pumpe hinein
- 284: Blutfluss aus der Pumpe heraus
- 285: Motorgehäuse
- 286: Wicklungen des Antriebs
- 287: Statormagnet des Antriebs
- 288: Pumpenlaufrad
- 289: Deckscheibe
- 290: Laufradschaufeln
- 291: Spülkanal im Laufradkörper
- 292: strömungsmechanische Radialstabilisierung
- 293: Abtriebsmagnete
- 294: seitlicher Strömungsraum zwischen Laufrad und Gehäuse
- 295: Pumpengehhäuse
- 296: hydrodynamisches Axiallager
- 297: Hochdruckbereich am Laufradaustritt
- 320: Pumpengehäuse
- 321: Pumpeneinlass
- 322: Pumpenauslass
- 323: Blutfluss in die Pumpe hinein
- 324: Blutfluss aus der Pumpe heraus
- 325: Statormagnet des Antriebs
- 326: Motorgehäuse
- 327: Wicklungen des Antriebs
- 328: Pumpenlaufrad
- 329: Deckscheibe
- 330: Laufradschaufeln
- 331: Spülkanal im Laufradkörper
- 332: strömungsmechanische Radialstabilisierung
- 333: Abtriebsmagnete
- 334: seitlicher Strömungsraum zwischen Laufrad und Gehäuse
- 335: Statormagnet des Antriebs
- 336: hydrodynamisches Axiallager
- 337: Hochdruckbereich am Laufradaustritt
- 400: Pumpengehäuse
- 401: Pumpeneinlass
- 402: Pumpenauslass
- 403: Pumpendeckel
- 404: Motorgehäuse
- 405: Kalotte
- 406: Elektromotor
- 407: Motorwelle
- 408: Polschuh
- 409: Antriebsmagnete
- 410: Abtriebsmagnete
- 411: Pumpenlaufrad
- 412: Laufradschaufeln
- 413: Spülkanal im Laufradkörper
- 414: Lagerkugel
- 415: Rotormagnet der permanentmagnetischen Lagerung
- 416: Statormagnet der permanentmagnetischen Lagerung
- 417: Blutfluss in die Pumpe hinein
- 418: Blutfluss aus der Pumpe heraus
- 419: Strömungskanal im Pumpeneinlass
- 419: Strömungskanal im Pumpenauslass
- 421: Ringkanal
- 422: Drehachse
- 425: Laufradvorderseite
- 426: Hochdruckbereich am Laufradaustritt Einmündung zum
- 427: Strömungsraum auf der Laufradrückseite
- 428: Hauptstrom
- 429: Spülstrom
- 430: Kalotte, Pivot-Rückseite
- 431: Pivot-Vorderseite
- 450: Pumpengehäuse
- 451: Pumpeneinlass
- 452: Pumpenauslass
- 453: Pumpendeckel
- 454: Motorgehäuse
- 455: Motordeckel, stationäres Pumpenelement
- 456: Elektromotor
- 457: Motorwelle
- 458: Polschuh
- 459: Antriebsmagnete
- 460: Abtriebsmagnete
- 461: Lagerkugel
- 462: Laufradschaufeln
- 463: Spülkanal im Laufradkörper
- 464: Lagerachse
- 465: Rotormagnet der permanentmagnetischen Lagerung
- 466: Statormagnet der permanentmagnetischen Lagerung
- 467: Blutfluss in die Pumpe hinein
- 468: Blutfluss aus der Pumpe heraus
- 469: Strömungskanal im Pumpeneinlass
- 470: Strömungskanal im Pumpenauslass
- 471: Ringkanal
- 473: Drehachse
- 475: Pumpenlaufrad
- 480: Laufradvorderseite
- 481: Hochdruckbereich am Laufradaustritt Einmündung zum
- 482: Strömungsraum auf der Laufradrückseite
- 483: zentraler Spülkanal
- 490: Hauptstrom
- 491: Spülstrom
- 495: Kalotte
- 496: Spitze des Motordeckels
- 500: Pumpengehäuse
- 501: Pumpeneinlass
- 502: Pumpenauslass
- 503: Pumpendeckel
- 504: Motorgehäuse
- 505: Motordeckel, stationäres Pumpenelement
- 506: Elektromotor
- 507: Motorwelle
- 508: Polschuh
- 509: Antriebsmagnete
- 510: Abtriebsmagnete
- 511: Lagerkugel
- 512: Laufradschaufeln
- 513: Spülkanal im Laufradkörper
- 514: Lagerachse
- 515: Rotormagnet der permanentmagnetischen Lagerung
- 516: Statormagnet der permanentmagnetischen Lagerung
- 517: Blutfluss in die Pumpe hinein
- 518: Blutfluss aus der Pumpe heraus
- 519: Strömungskanal im Pumpeneinlass
- 520: Strömungskanal im Pumpenauslass
- 521: Ringkanal
- 524: Deckscheibe
- 525: Pumpenlaufrad
- 530: Kalotte
- 531: Spitze des Motordeckels
- 532: Laufradvorderseite
- 533: Hochdruckbereich am Laufradaustritt Einmündung zum
- 534: Strömungsraum auf der Laufradrückseite
- 535: zentraler Spülkanal
- 536: Ausmündung der Leckageströmung
- 540: Hauptstrom
- 541: Spülstrom
- 542: Leckageströmung
- 600: Pumpengehäuse
- 601: Pumpeneinlass
- 602: Pumpenauslass
- 603: Pumpendeckel
- 604: Motorgehäuse
- 605: Motordeckel, stationäres Pumpenelement
- 606: Elektromotor
- 607: Motorwelle
- 608: Polschuh
- 609: Antriebsmagnete
- 610: Abtriebsmagnete
- 611: Lagerkugel
- 612: Laufradschaufeln
- 613: Spülkanal im Laufradkörper
- 614: Lagerachse
- 617: Blutfluss in die Pumpe hinein
- 618: Blutfluss aus der Pumpe heraus
- 619: Strömungskanal im Pumpeneinlass
- 620: Strömungskanal im Pumpenauslass
- 621: Ringkanal
- 624: Deckscheibe
- 625: Pumpenlaufrad
- 630: Kalotte
- 631: Spitze des Motordeckels
- 632: Laufradvorderseite
- 633: Hochdruckbereich am Laufradaustritt Einmündung zum
- 634: Strömungsraum auf der Laufradrückseite
- 635: zentraler Spülkanal
- 636: Ausmündung der Leckageströmung
- 640: Hauptstrom
- 641: Spülstrom
- 642: Leckageströmung
- 645: Drehachse
- 660: Rückstellkraft zwischen Antriebmagneten und Abtriebsmagneten im verkleinerten Luftspalt
- 661: Rückstellkraft zwischen Antriebmagneten und Abtriebsmagneten im vergrößerten Luftspalt
- 662: Rückstellmoment auf Abtriebsmagnete bei Verkippungen des Laufrades
- 663: Auslenkmoment auf Abtriebsmagnete bei Verkippungen des Laufrades
- 670: Erster Abtriebsmagnet bei Teilung der Kupplungsmagnetsegmente in radialer Richtung
- 671: Zweiter Abtriebsmagnet bei Teilung der Kupplungsmagnetsegmente in radialer Richtung
- 672: Erster Antriebsmagnet bei Teilung der Kupplungsmagnetsegmente in radialer Richtung
- 673: Zweiter Antriebsmagnet bei Teilung der Kupplungsmagnetsegmente in radialer Richtung
- 680: Rückstellkraft zwischen Antriebmagneten und Abtriebsmagneten im verkleinerten Luftspalt bei Teilung der Kupplungsmagnetsegmente
- 681: Rückstellkraft zwischen Antriebmagneten und Abtriebsmagneten im vergrößerten Luftspalt bei Teilung der Kupplungsmagnetsegmente
- 682: Rückstellmoment auf geteilte Abtriebsmagnete bei Verkippungen des Laufrades
- 683: Auslenkmoment auf geteilte Abtriebsmagnete bei Verkippungen des Laufrades
- 900: Elliptischer Teil des Pumpengehäuses
- 901: Kreiszylindrischer Teil des Pumpengehäuses
- 902: Pumpeneinlass
- 903: Pumpenauslass
- 904: Gehäusedeckel
- 905: Pumpenlaufrad
- 906: Zuströmkanal
- 910: Elliptischer Teil der Antriebseinheit
- 911: Kreiszylindrischer Teil der Antriebseinheit
- 930: Rotormagnet bei einem 2-poligen elektromagnetischen Antrieb
- 931: Statormagnet bei einem 2-poligen elektromagnetischen Antrieb mit elliptischem Querschnitt
- 932: Wicklungen bei einem 2-poligen elektromagnetischen Antrieb mit elliptischem Querschnitt
- 940: Rotormagnet bei einem 4-poligen elektromagnetischen Antrieb mit elliptischem Querschnitt
- 941: Statormagnet bei einem 4-poligen elektromagnetischen Antrieb mit elliptischem Querschnitt
- 942: Wicklungen bei einem 4-poligen elektromagnetischen Antrieb mit elliptischem Querschnitt
- 1000: Blutpumpe als trennbarer Pumpeneinheit
- 1020: Drehverschluss an der Pumpeneinheit
- 1060: Antriebsgehäuse
- 1061: Kühlrippen
- 1062: Drehverschluss an der Antriebseinheit
- 1063: Motordeckel
- 1064: Antriebsdeckel
- 1070: Antriebsmagnete
- 1071: Polschuh
- 1072: Motorwelle
- 1100: Blutpumpe als trennbarer Pumpeneinheit
- 1120: Drehverschluss an der Pumpeneinheit
- 1160: Antriebsgehäuse
- 1161: Sauererstoffeinlass
- 1162: Sauererstoffauslass
- 1163: Antriebsdeckel
- 1164: Drehverschluss an der Antriebseinheit
- 1170: Gasturbine
- 1171: Turbinenschaufeln
- 1172: Antriebswelle
- 1173: Drehachse
- 1174: Erstes Wälzlager
- 1175: Zweites Wälzlager
- 1176: Erste Stützplatte
- 1177: Zweite Stützplatte
- 1178: Polschuh
- 1179: Antriebsmagnete
- 1180: Sauerdurchströmte Innenraum der Antriebseinheit
- 1181: Einlassseitiger Strömungskanal
- 1182: Auslassseitiger Strömungskanal
- 1190: Eintretender Sauerstoff
- 1191: Sauerstoffströmung innerhalb der Antriebseinheit
- 1192: Austretender Sauerstoff
- 2000: Blutpumpe
- 2010: Turbine
- 2020: Magnetkupplung
- 2030: Drosselventil
- 2040: Sauerstoffbehälter
- 2050: Kompressor
- 2060: Patient
- 2100: Blutpumpe
- 2110: Turbine
- 2120: Magnetkupplung
- 2130: Drosselventil
- 2140: Sauerstoffbehälter
- 2150: Kompressor
- 2160: Patient
- 2170: Oxygenator
- 2180: Drosselventil

## Patentansprüche

1. Blutpumpe mit einem Laufrad (12) mit einer Drehachse (23) in einem Pumpengehäuse (1), wobei die Blutpumpe einen Einlass (2) und einen Auslass (3) aufweist und sie mindestens zwei räumlich voneinander getrennte Elemente enthält, **dadurch gekennzeichnete dass** wenigstens eines der Elemente Lomakin-Lager darstellt und dass mindestens ein weiteres Element (19,20) ein radiales Magnetlager darstellt, wobei die Blutpumpe eine Axiallagerung des Laufrades (12) aufweist, welche auf strömungsmechanischen Kräften basiert, wobei dies durch ein hydrodynamisches Axiallager auf der Laufradrückseite(259) erreicht wird und ein zentraler Spülkanal (248) im Laufradkörper (234) vorgesehen ist, mit dem an der Laufradvorderseite (254) ein Spülstrom (251, 252) vom Hauptstrom (250) abgezweigt wird und über den zentralen Spülkanal (248) im Laufradkörper (234) auf die Laufradrückseite (259) geleitet wird, wobei eine auf der Rückseite des Laufrades (234) vorgesehene sekundäre Beschaufelung (261, 206) bei Drehbewegungen des Laufrades (234) im Betrieb der Blutpumpe bewirkt, dass die Spülstromung (252) infolge der einwirkenden Zentrifulgaleffekte über Spülkanäle (260) radial nach außen befördert wird und von dort wieder in die Hauptströmung (250) mündet, wobei ferner die Spülströmung (252) gleichzeitig als Lagerströmung für ein hydrodynamisches Axiallager (262, 240) auf der Laufradrückseite (259) verwendet wird, wobei ferner das radiale Magnetlager durch eine Magnetkupplung (235, 236, 237, 238) gebildet wird.

2. Blutpumpe nach Anspruch 1, **dadurch gekennzeichnet, dass** das Lomakin-Lager durch das Pumpengehäuse (1) und ein Pumpenlaufrad (12) und/oder einen Bestandteil des Pumpenlaufrades (12) gebildet wird.

3. Blutpumpe nach Anspruch 1 oder 2, **dadurch gekennzeichnet, dass** das radiale Magnetlager ein elektromagnetisches Radiallager ist.

4. Blutpumpe nach Anspruch 1 oder 2, **dadurch gekennzeichnet, dass** das radiale Magnetlager ein permanentmagnetisches Radiallager ist.

5. Blutpumpe nach einem der vorangehenden Ansprüche, **dadurch gekennzeichnet, dass** das radiale Magnetlager mindestens zwei Ringmagnete beinhaltet.

6. Blutpumpe nach Anspruch 5, **dadurch gekennzeichnet, dass** wenigstens einer der Ringmagnete in dem Pumpenlaufrad (12) und/oder in einen der Bestandteile des Pumpenlaufrades (12) integriert ist.

7. Blutpumpe nach einem der vorangehenden Ansprüche, **dadurch gekennzeichnet, dass** das radiale Magnetlager durch abstoßende magnetische Kräfte wirkt.

8. Blutpumpe nach einem der Ansprüche 1 bis 6 **dadurch gekennzeichnet, dass** das radiale Magnetlager durch anziehende magnetische Kräfte wirkt.

9. Blutpumpe nach einem der Ansprüche 4 bis 8, **dadurch gekennzeichnet, dass** das radiale Magnetlager als separates permanentmagnetisches Radiallager ausgebildet ist.

10. Blutpumpe nach einem der Ansprüche 4 bis 8, **dadurch gekennzeichnet, dass** das radiale Magnetlager als separates elektromagnetisches Radiallager ausgebildet ist.

11. Blutpumpe nach Anspruch 10, **dadurch gekennzeichnet, dass** das radiale Magnetlager einen Rotormagneten im Laufrad (12) und einen Statormagneten im Pumpengehäuse (1) beinhaltet.

12. Blutpumpe nach Anspruch 1, **dadurch gekennzeichnet, dass** die magnetische Kupplung eine axiale Magnetkupplung ist.

13. Blutpumpe nach Anspruch 1, **dadurch gekennzeichnet, dass** die magnetische Kupplung eine diagonale Magnetkupplung ist.

14. Blutpumpe nach einem der vorangehenden Ansprüche, **dadurch gekennzeichnet, dass** das radiale Magnetlager geregelte elektromagnetische Kräfte ausübt.

15. Blutpumpe nach einem der vorangehenden Ansprüche, **dadurch gekennzeichnet, dass** das Lomakin-Lager zwischen Laufrad (12) und dem Pumpengehäuse (1) wirkt.

16. Blutpumpe nach einem der vorangehenden Ansprüche, **dadurch gekennzeichnet, dass** das Lomakin-Lager zwischen einer mit den Laufradschaufeln (13) verbundenen Deckscheibe (18) und dem Pumpengehäuse (1) wirkt.

17. Blutpumpe nach einem der vorangehenden Ansprüche, **dadurch gekennzeichnet, dass** das Lomakin-Lager zwischen Laufradschaufeln (13) und dem Pumpengehäuse (1) wirkt.

## Claims

1. A blood pump with an impeller (12) with an axis of rotation (23) in a pump housing (1), the blood pump having an inlet (2) and an outlet (3) and containing at least two spatially separated elements, **characterized in that** at least one of the elements is a Lomakin bearing and that at least one further element (19, 20) is a radial magnetic bearing, the blood pump has an axial bearing of the impeller (12), which is based on fluidic forces, wherein this is achieved by a hydrodynamic axial bearing on the impeller back side (259) and a central flushing channel (248) is provided in the impeller body (234), with which a flushing flow (251, 252) is branched off from the main flow (250) at the impeller front side (254) and is conducted via the central flushing channel (248) in the impeller body (234) to the impeller back side (259), wherein a secondary blading (261, 206) is provided on the back side of the impeller (234) and has the effect due to the rotary movements of the impeller (234) during operation of the blood pump that the flushing flow (262) is directed radially outwards via flushing channels (260) as a result of the centrifugal effects acting on it and from there flows back into the main flow (250), whereby furthermore the flushing flow (252) simultaneously is used as a bearing flow for a hydrodynamic thrust bearing (262, 240) on the back of the impeller (259), wherein further the radial magnetic bearing is formed by a magnetic coupling (235, 236, 237, 238).

2. The blood pump according to Claim 1, **characterized in that** the Lomakin bearing is formed by the pump housing (1) and by a pump impeller (12) and/or by a component of the pump impeller (12).

3. The blood pump according to Claim 1 or 2, **characterized in that** the radial magnetic bearing is an electromagnetic radial bearing.

4. The blood pump according to Claim 1 or 2, **characterized in that** the radial magnetic bearing is a permanent magnetic radial bearing.

5. The blood pump according to any of the preceding claims, **characterized in that** the radial magnetic bearing comprises at least two annular magnets.

6. The blood pump according to Claim 5, **characterized in that** at least one of the annular magnets is integrated into the pump impeller (12) and/or into one of the components of the pump impeller (12).

7. The blood pump according to any of the preceding claims, **characterized in that** the radial magnetic bearing functions by means of repulsive magnetic forces.

8. The blood pump according to any of Claims 1 to 6, **characterized in that** the radial magnetic bearing functions by means of attractive magnetic forces.

9. The blood pump according to any of Claims 4 to 8, **characterized in that** the radial magnetic bearing is configured as a separate permanent magnetic radial bearing.

10. The blood pump according to any of Claims 4 to 8, **characterized in that** the radial magnetic bearing is configured as a separate electromagnetic radial bearing.

11. The blood pump according to Claim 10, **characterized in that** the radial magnetic bearing has a rotor magnet in the impeller (12) and a stator magnet in the pump housing (1).

12. The blood pump according to Claim 1, **characterized in that** the magnetic coupling is an axial magnetic coupling.

13. The blood pump according to Claim 1, **characterized in that** the magnetic coupling is a diagonal magnetic coupling.

14. The blood pump according to any of the preceding claims, **characterized in that** the radial magnetic bearing exerts regulated electromagnetic forces.

15. The blood pump according to any of the preceding claims, **characterized in that** the Lomakin bearing is operational between the impeller (12) and the pump housing (1).

16. The blood pump according to any of the preceding claims, **characterized in that** the Lomakin bearing is operational between a shroud (18) connected with impeller blades (13) and the pump housing (1).

17. The blood pump according to any of the preceding claims, **characterized in that** the Lomakin bearing is operational between the impeller blades (13) and the pump housing (1).

## Revendications

1. Pompe à sang avec une roue (12) avec un axe de rotation (23) dans un boîtier de pompe (1), la pompe à sang comportant une entrée (2) et une sortie (3) et contenant au moins deux éléments séparés l'un de l'autre dans l'espace, **caractérisée en ce qu**'au moins l'un des éléments tient lieu de palier de Lomakin et en ce qu'au moins un autre élément (19, 20) tient lieu de palier magnétique radial, dans lequel la pompe à sang présente un palier axial de la roue (12), qui est basé sur des forces fluidiques, ce qui est obtenu par un palier axial hydrodynamique sur la face arrière de la roue (259) et un canal de rinçage central (248) est prévu dans le corps de la roue (234) par le front (254) de la roue, avec lequel un flux de rinçage (251, 252) est dérivé du flux principal (250) et est conduit via le canal de rinçage central (248) dans le corps de la roue (234) vers la face arrière de la roue (259), dans lequel une aubage secondaire (261, 206) prévue à l'arrière de la roue (234) lors des mouvements de rotation de la roue (234) pendant le fonctionnement de la pompe à sang a pour effet que le flux de rinçage (262) est dirigé radialement vers l'extérieur par des canaux de rinçage (260) en raison des effets centrifuges qui agissent sur lui et, de là, retourne dans le flux principal (250), où en outre le flux de rinçage (252) simultanément comme flux de palier pour un palier de butée hydrodynamique (262, 240) est utilisé à l'arrière de la roue (259), où en outre le palier magnétique radial est formé par un accouplement magnétique (235, 236, 237, 238).

2. Pompe à sang selon la revendication 1, **caractérisée en ce que** le palier de Lomakin est constitué par le boîtier de la pompe (1) et une roue de pompe (12) et/ou un composant de la roue de pompe (12).

3. Pompe à sang selon la revendication 1 ou 2, **caractérisée en ce que** le palier magnétique radial est un palier radial électromagnétique.

4. Pompe à sang selon la revendication 1 ou 2, **caractérisée en ce que** le palier magnétique radial est un palier radial à magnétisme permanent.

5. Pompe à sang selon l'une des revendications précédentes, **caractérisée en ce que** le palier magnétique radial contient au moins deux aimants annulaires.

6. Pompe à sang selon la revendication 5, **caractérisée en ce qu'**au moins l'un des aimants annulaires est intégré dans la roue de la pompe (12) et/ou dans l'un des composants de la roue de la pompe (12).

7. Pompe à sang selon l'une des revendications précédentes, **caractérisée en ce que** le palier magnétique radial agit par des forces magnétiques répulsives.

8. Pompe à sang selon l'une des revendications 1 à 6, **caractérisée en ce que** le palier magnétique radial agit par des forces magnétiques attractives.

9. Pompe à sang selon l'une des revendications 4 à 8, **caractérisée en ce que** le palier magnétique radial se présente sous la forme d'un palier radial séparé à magnétisme permanent.

10. Pompe à sang selon l'une des revendications 4 à 8, **caractérisée en ce que** le palier magnétique radial se présente sous la forme d'un palier radial électromagnétique séparé.

11. Pompe à sang selon la revendication 10, **caractérisée en ce que** le palier magnétique radial contient un aimant de rotor dans la roue (12) et un aimant de stator dans le boîtier de la pompe (1).

12. La pompe à sang selon la revendication 1, **caractérisée en ce que** le couplage magnétique est un couplage magnétique axial.

13. Pompe à sang selon la revendication 1, **caractérisée en ce que** le couplage magnétique est un couplage magnétique diagonal.

14. Pompe à sang selon l'une des revendications précédentes, **caractérisée en ce que** le palier magnétique radial exerce des forces électromagnétiques réglées.

15. Pompe à sang selon l'une des revendications précédentes, **caractérisée en ce que** le palier de Lomakin agit entre la roue (12) et le boîtier de la pompe (1).

16. Pompe à sang selon l'une des revendications précédentes, **caractérisée en ce que** le palier de Lomakin agit entre un disque de recouvrement (18) relié aux aubes (13) de la roue (12) et le boîtier de la pompe (1).

17. Pompe à sang selon l'une des revendications précédentes, **caractérisée en ce que** le palier de Lomakin agit entre les aubes (13) de la roue (12) et le boîtier de la pompe (1).
